# EUROPEAN PATENT SPECIFICATION

(11) **EP 4 181 774 B1**
(45) Date of publication and mention of the grant of the patent: **08.10.2025**
(21) Application number: 21841393.8
(22) Date of filing: 21.05.2021
(51) Int. Cl.: A61B 5/00, A61N 1/36, A61N 1/362, A61M 16/00, A61M 16/06, A61M 16/10

(54) **DEVICE FOR PREVENTING SUDDEN DEATH AND PROTECTING OXYGEN STATUS DURING SEIZURE ACTIVITY**
VORRICHTUNG ZUR VERHINDERUNG VON PLÖTZLICHEM TOD UND ZUM SCHUTZ DES SAUERSTOFFSTATUS WÄHREND EINER ANFALLSAKTIVITÄT
DISPOSITIF DE PRÉVENTION DE LA MORT SUBITE ET DE PROTECTION DE L'ÉTAT D'OXYGÈNE PENDANT UNE ACTIVITÉ DE CRISE D'ÉPILEPSIE

(30) Priority: 14.07.2020 US 202063051448 P
(43) Date of publication of application: 24.05.2023
(73) Proprietor: The Research Foundation for The State University of New York, Albany, New York 12207 (US)
(72) Inventor: STEWART, Mark, East Hanover, New Jersey 07936 (US); KOLLMAR, Richard, Bronx, New York 10463 (US)
(74) Representative: Dennemeyer & Associates S.A.
(86) International application number: PCT/US2021/033590
(87) International publication number: WO 2022/015414

(56) References cited:
- WO-A1-2015/193672
- US-A- 5 199 423
- US-A1- 2004 112 211
- US-A1- 2010 198 289
- US-A1- 2012 310 050
- US-A1- 2018 326 171

## Description

### FIELD

The present disclosure relates to devices and methods for preventing sudden death and protecting oxygen status during seizure activity.

### BACKGROUND

Sudden death in epilepsy (SUDEP) is the most common cause of death among persons with epilepsy (Devinsky et al., "Sudden Unexpected Death in Epilepsy: Epidemiology, Mechanisms, and Prevention," Lancet Neural. 15(10):1075-88 (2016)) and most of those deaths occur at night when the individual is in bed (Purnell et al., "Dead in the Night: Sleep-Wake and Time-Of-Day Influences on Sudden Unexpected Death in Epilepsy," Front Neurol. 9:1079 (2018)).

It was previously shown that epileptic seizure activity can spread through lower brain areas to cause laryngospasm sufficient to completely obstruct the airway (obstructive apnea) and that the period of obstructive apnea can last long enough for respiratory arrest to occur (Nakase et al., "Laryngospasm, Central and Obstructive Apnea During Seizures: Defining Pathophysiology For Sudden Death in a Rat Model," Epilepsy Res. 128:126-139 (2016); Stewart et al., "Obstructive Apnea Due to Laryngospasm Links Ictal to Postictal Events in SUDEP Cases and Offers Practical Biomarkers for Review of Past Cases and Prevention of New Ones," Epilepsia 58:e87-e90 (2017)). Respiratory arrest includes, for example, the point at which the individual experiencing seizure activity stops attempting to draw a breath. This is considered to be equivalent to the "onset" of terminal apnea as defined by Ryvlin et al. (Ryvlin et al., "Incidence and Mechanisms of Cardiorespiratory Arrests in Epilepsy Monitoring Units (MORTEMUS): A Retrospective Study," Lancet Neural. 12:966-77 (2013)).

Associated with the period of airway occlusion is a progressive loss of oxygen from the blood (desaturation) and this loss of oxygen can contribute to seizure termination (Hotta et al., "Vagus Nerve Stimulation-Induced Bradyarrhythmias in Rats," Auton. Neurosci. 151:98-105 (2009); Nakase et al., "Laryngospasm, Central and Obstructive Apnea During Seizures: Defining Pathophysiology For Sudden Death in a Rat Model," Epilepsy Res. 128:126-139 (2016); Stewart et al., "Obstructive Apnea Due to Laryngospasm Links Ictal to Postictal Events in SUDEP Cases and Offers Practical Biomarkers for Review of Past Cases and Prevention of New Ones," Epilepsia 58:e87-e90 (2017)). As the seizure ends, on its own or from hypoxia, it also ceases to be a stimulus for laryngospasm, and the airway can open. If respiratory arrest occurs before the airway reopens, the individual will progress from respiratory arrest to cardiac arrest and death unless mechanically resuscitated by another individual. If the airway reopens before respiratory arrest occurs, the individual will spontaneously resume breathing and recover from the event.

In mouse studies, DBA/2 mice experiencing audiogenic seizures that would normally be fatal with nearly 100% mortality were protected by experiencing those seizures in an oxygen atmosphere (Venit et al., "Oxygenation Prevents Sudden Death in Seizure-Prone Mice," Epilepsia 45:993-6 (2004) and Willott et al., "Roles of Anoxia and Noise-Induced Hearing Loss in the Postictal Refractory Period for Audiogenic Seizures in Mice," J. Comp. Physiol. Psychol. 90:373-81 (1976)). Similarly, selective serotonergic reuptake inhibitors (SSRIs) such as fluoxetine have been shown to protect against death in multiple strains of audiogenic seizure prone mice (Faingold et al., "Serotonin and Sudden Death: Differential Effects of Serotonergic Drugs on Seizure-Induced Respiratory Arrest in DBA/1 mice," Epilepsy Behav. 37C:198-203 (2014); Faingold et al., "Prevention of Seizure-Induced Sudden Death in a Chronic SUDEP Model by Semichronic Administration of a Selective Serotonin Reuptake Inhibitor," Epilepsy Behav. 22:186-90 (2011) and Zeng et al., "Fluoxetine Prevents Respiratory Arrest Without Enhancing Ventilation in DBA/1 Mice," Epilepsy Behav. 45:1-7 (2015)) suggesting that these drugs protect the brainstem circuits critical for respiration (Bateman et al., "Serotonin Reuptake Inhibitors are Associated With Reduced Severity of Ictal Hypoxemia in Medically Refractory Partial Epilepsy," Epilepsia 51:2211-14 (2010); Blum, A.S., "Respiratory Physiology of Seizures," J. Clin. Neurophysiol. 26:309-15 (2009); Faingold et al., "Serotonin and Sudden Death: Differential Effects of Serotonergic Drugs on Seizure-Induced Respiratory Arrest in DBA/1 mice," Epilepsy Behav. 37C:198-203 (2014); Faingold et al., "Differences in Serotonin Receptor Expression in the Brainstem May Explain the Differential Ability of a Serotonin Agonist to Block Seizure-Induced Sudden Death in DBA/2 vs. DBA/1 M," Brain Res. 1418:104-10 (2011); Feng et al., "Abnormalities of Serotonergic Neurotransmission in Animal Models of SUDEP," Epilepsy Behav. 71:174-80 (2015); Richerson et al., "The Serotonin Axis: Shared Mechanisms in Seizures, Depression, and SUDEP," Epilepsia 52(Suppl 1):28-38 (2011); Tupal et al., "Evidence Supporting a Role of Serotonin in Modulation of Sudden Death Induced by Seizures in DBA/2 Mice," Epilepsia 47:21-6 (2006); and Uteshev et al., "Abnormal Serotonin Receptor Expression in DBA/2 Mice Associated With Susceptibility to Sudden Death Due to Respiratory Arrest," Epilepsy Res. 88:183-188 (2010)).

While partial airway closure due to seizure induced laryngospasm is common (Nakase et al., "Laryngospasm, Central and Obstructive Apnea During Seizures: Defining Pathophysiology For Sudden Death in a Rat Model," Epilepsy Res. 128:126-139 (2016)), only a small fraction of human epileptic seizures include periods of complete airway closure and high risk for sudden death. However, there is no current approach to differentiating the high risk from the low risk seizures.

As the amount, quality, and range of evidence increases from cases of SUDEP, near miss cases, and preclinical models, more focused ideas about the underlying mechanism(s) and potential biomarkers to help stratify risk are being proposed (*see e.g.,* Devinsky et al., "Sudden Unexpected Death in Epilepsy: Epidemiology, Mechanisms, and Prevention," Lancet Neural. 15(10):1075-88 (2016); Jansen, K. et al., "Cardiac changes in epilepsy," Seizure 19:455-60 (2010); Lacuey et al. "The Incidence and Significance of Periictal Apnea in Epileptic Seizures," Epilepsia 59:573-582 (2018); Poh et al., "Autonomic Changes With Seizures Correlate With Postictal EEG Suppression," Neurology 78:1868-76 (2012); Stewart et al., "Obstructive Apnea Due to Laryngospasm Links Ictal to Postictal Events in SUDEP Cases and Offers Practical Biomarkers for Review of Past Cases and Prevention of New Ones," Epilepsia 58:e87-e90 (2017); Vilella et al., "Postconvulsive Central Apnea as a Biomarker for Sudden Unexpected Death in Epilepsy (SUDEP)," Neurology 92:e171-e182 (2018)).

SUDEP is a rare occurrence, but is the most common cause of death among persons with epilepsy (e.g., Barot et al., "Autonomic Aspects of Sudden Unexpected Death in Epilepsy (SUDEP)," Clin. Auton. Res. 29:151-160 (2019); Devinsky et al., "Sudden Unexpected Death in Epilepsy: Epidemiology, Mechanisms, and Prevention," Lancet Neural. 15:1075-88 (2016)) and most deaths occur at night when the individual is in bed (e.g., Purnell et al., "Dead in the Night: Sleep-Wake and Time-Of-Day Influences on Sudden Unexpected Death in Epilepsy," Front Neurol. 9:1079 (2018); Clark et al., "A Population-Based Post Mortem Study of Sudden Unexpected Death in Epilepsy," J. Clin. Neurosci. 23:58-62 (2016); Massey et al., "Mechanisms of Sudden Unexpected Death in Epilepsy: The Pathway to Prevention," Nat. Rev. Neurol. 10:271-82 (2014); Ryvlin et al., "Incidence and Mechanisms of Cardiorespiratory Arrests in Epilepsy Monitoring Units (MORTEMUS): A Retrospective Study," Lancet Neurol. 12:966-77 (2013); and Shorvon et al., "Sudden Unexpected Death in Epilepsy," Lancet 378:2028-38 (2011)). Much of the challenge in collecting clinical data comes from the rarity of actual deaths and the unlikely occurrence of detailed physiological records during seizure events when individuals are out of the hospital.

Patent document US2012/310050A1 is hereby acknowledged.

The present disclosure is directed to protection of oxygen status during seizure activity.

### SUMMARY

The invention is defined by the appended claims.

A first aspect relates to a system for prevention of sudden death. The system includes a wearable seizure detection device; a base unit; and a computer, the computer configured to receive input from the seizure detection device and trigger the base unit to release oxygen under a condition effective to enrich an environmental oxygen level and prevent sudden death when the input indicates a seizure.

In one embodiment, oxygen is released within about 30 seconds after a seizure is detected by the seizure detection device. In one embodiment, the condition is effective to enrich an environmental oxygen level to at least 50%. In another embodiment, the oxygen is released under sustained conditions. In yet another embodiment, oxygen is released for a fixed duration of time. In one embodiment, the fixed duration of time is for a period of between about 30 seconds to about 5 minutes.

In one embodiment, the base unit further includes an oxygen tank; a gas regulator; an electronic valve; a flow meter; and a nozzle. In one embodiment, the nozzle is attached to the base unit by a flexible tubing. In one embodiment, the flexible tubing has a length of more than about 1 foot. In another embodiment, the base unit further includes an attachment device capable of providing a concentrated environmental oxygen level surrounding the attachment device. In one embodiment, the attachment device is a cone or a canopy. In another embodiment, the attachment device is a conical canopy.

In one embodiment, the base unit further includes an oxygen sensor. In another embodiment, oxygen is released at a flow of between about 1 and about 250 standard cubic feet per minute. In yet another embodiment, the oxygen is released at a pressure of between about 1 psi and about 100 psi. In one embodiment, at least one of the input and the trigger is transmitted wirelessly. In another embodiment, the system further includes a remote control, the remote control capable of providing on demand oxygen release.

A second aspect relates to a method of preventing sudden death. The method includes enriching an environmental oxygen level for a sleeping subject using the system described herein.

In one embodiment, the seizure disorder is selected from the group consisting of epilepsy and any neurological or other medical condition that is associated with generalized seizures, absence seizures, tonic-clonic seizures, focal seizures, simple focal seizures, complex focal seizures, and secondary generalized seizures.

A third aspect relates to a portable system for prevention of sudden death. The portable system includes an oronasal mask; a compressed oxygen cartridge, wherein the compressed oxygen cartridge is coupled to the oronasal mask; and a fastener, wherein the fastener is coupled to the oronasal mask, wherein the system releases oxygen upon activation under a condition effective to enrich an environmental oxygen level and prevent sudden death.

In one embodiment, the oronasal mask is flexible. In another embodiment, the oronasal mask is collapsible. In one embodiment, the fastener is an elastic strap. In one embodiment, the compressed oxygen cartridge is at least about 5 grams. In one embodiment, the system further includes one or more additional compressed oxygen cartridges. In one embodiment, the condition is effective to enrich an environmental oxygen level to at least 40%. In one embodiment, oxygen is released under sustained conditions. In one embodiment, oxygen is released for a fixed duration of time. In one embodiment, the fixed duration of time is for a period of between about 30 seconds to about 5 minutes.

In one embodiment, the system further includes a flow indicator. In another embodiment, the system further includes a flow regulator. In one embodiment, the oxygen cartridge is coupled to the oronasal mask by a flexible tubing. In one embodiment, the flexible tubing has a length of less than about 10 feet.

In one embodiment, the system further includes an oxygen sensor. In one embodiment, oxygen is released at a flow of between about 1 and about 25 liters per minute. In one embodiment, the oxygen is released at a pressure of between about 1 psi and about 100 psi. In one embodiment, the system further includes a storage pouch. In one embodiment, the system further includes an armband. In one embodiment, the system further includes a wearable seizure detection device.

A fourth aspect relates to a method of preventing sudden death, the method including enriching an environmental oxygen level for a subject using the portable system for prevention of sudden death described herein, wherein the subject has a seizure disorder.

In one embodiment, the seizure disorder is selected from the group consisting of epilepsy and or any neurological or other medical condition that is associated with generalized seizures, absence seizures, tonic-clonic seizures, focal seizures, simple focal seizures, complex focal seizures, and secondary generalized seizures.

### BRIEF DESCRIPTION OF THE DRAWINGS

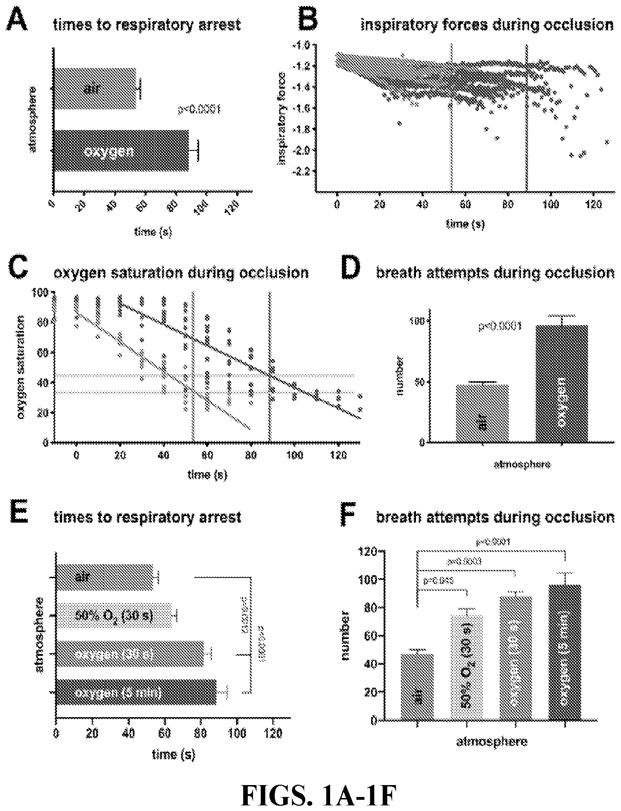

FIGS. 1A-1F show the impact of oxygen exposure on times to respiratory arrest in a rat model of complete airway occlusion. In FIG. 1A, rats exposed to room air experienced respiratory arrest about 50 seconds after the onset of controlled airway occlusion (a method to simulate laryngospasm-based complete airway closure). By contrast, rats breathing in an oxygen enriched atmosphere lasted nearly twice as long before respiratory arrest occurred. FIG. 1B shows a plot of each attempted breath and its associated inspiratory force. Animals breathing air are shown in red and animals breathing oxygen are shown in blue. The large increases in inspiratory effort just before respiratory arrest are seen near the mean times for respiratory arrest (vertical red and blue lines). Because large inspiratory effort against a closed airway is biophysically favorable for the generation of pulmonary edema, the persistence of smaller inspiratory breath attempts protects longer against the development of pulmonary edema. FIG. 1C shows that oxygen shifts the desaturation profile to later times. There is nearly a 40 second delay in desaturation onset in animals breathing oxygen versus those breathing air. This delay in the onset of desaturation and the time for respiratory arrest is the basis of the protection against death. The gray horizontal lines indicate the intersection of the linear regression line with the mean time to respiratory arrest. FIG. 1D shows that the total number of breath attempts made during complete airway closure is significantly greater in animals that breathed oxygen prior to airway occlusion. This is another representation of the protective role of oxygen. FIG. 1E shows a comparison of briefer oxygen exposure times and lower concentrations of oxygen on the times to respiratory arrest. Brief (30 second) exposure to oxygen is as protective as longer (5 minute) exposures. Brief exposure to 50% oxygen did not retain statistical significance when compared to air after post hoc correction for multiple comparisons (because the group sizes were small), but in a straight t-test comparing only air and 50% oxygen the time to respiratory arrest was prolonged (p=0.025). Mean times were: 53.6 (air), 63.9 (brief 50% 02), 81.6 (brief O2), and 88.4 (oxygen) seconds. FIG. 1F shows breath attempts for all impact of brief and 50% oxygen. P values shown for the isolated pairs of bars in panels FIG. 1A and FIG. 1D are after post-hoc correction for multiple comparisons.

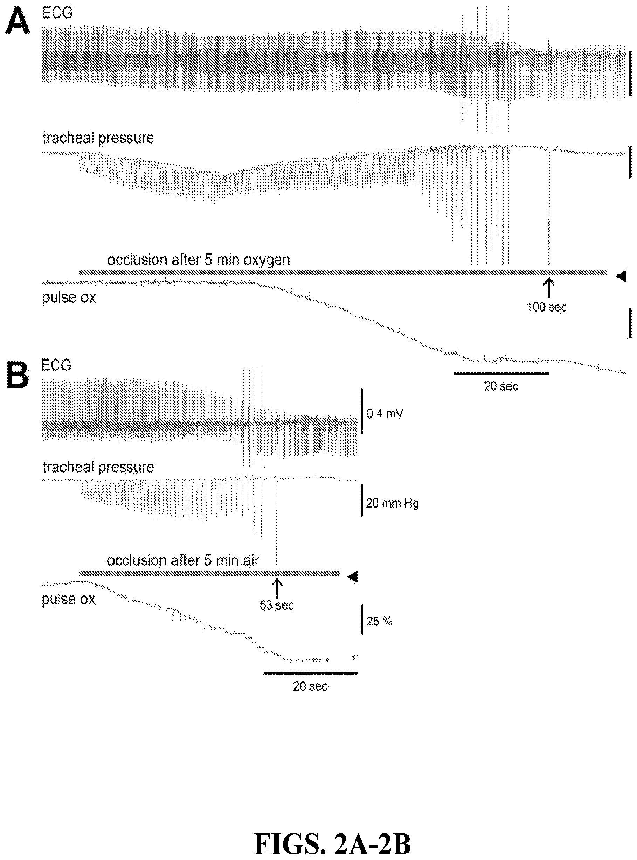

FIGS. 2A-2B show examples of prolongation of the time to respiratory arrest from oxygen exposure. FIG. 2A shows controlled airway occlusion after breathing oxygen for five minutes. Time of occlusion is marked by heavy horizontal line. Last breath attempt (i.e. the point of respiratory arrest) occurred at 100 seconds after occlusion onset (marked by arrow). Top trace shows limb lead ECG with initial normal sinus rhythm that showed significant ST segment elevation indicative of hypoxic changes in the heart, bradycardia, and premature ventricular contractions starting at about 80 seconds post-occlusion. The forces associated with breath attempts during airway occlusion are shown in the center trace, and the pulse oximeter output is shown in the bottom trace of the panel. One hundred percent oxygen saturation is marked by the arrowhead at the right edge of the panel and coincides with the bottom of the horizontal bar marking the period of occlusion. FIG. 2B is similar to FIG. 2A, but from an animal that was breathing air. Note the significantly shorter time to respiratory arrest (53 seconds) and shorter times to significant cardiac rhythm irregularities (about 40 seconds). Calibrations are indicated on the figure.

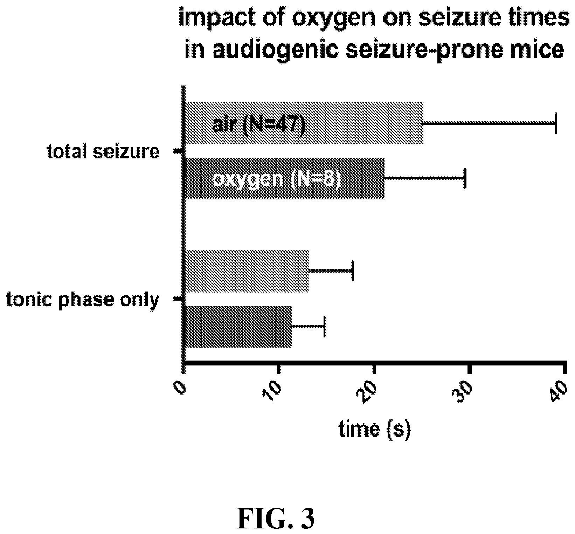

FIG. 3 shows that oxygen does not prolong the total seizure time, nor the time spent in the tonic hindlimb extension phase. In audiogenic seizure-prone mice, seizures are stereotyped and consist of a running phase, a clonic phase, and a tonic phase that included hindlimb extension. Mice that do not reach the tonic hindlimb extension phase do not die. The hindlimb extension phase and the total seizure times are unchanged by oxygen, but the time to respiratory arrest is prolonged.

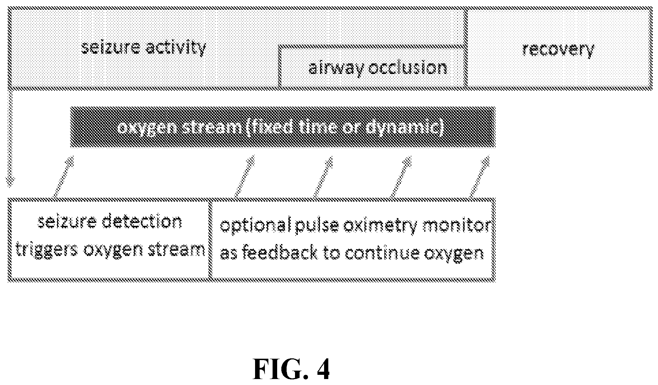

FIG. 4 shows a diagram of core elements for the prevention of death in epileptic individuals during sleep.

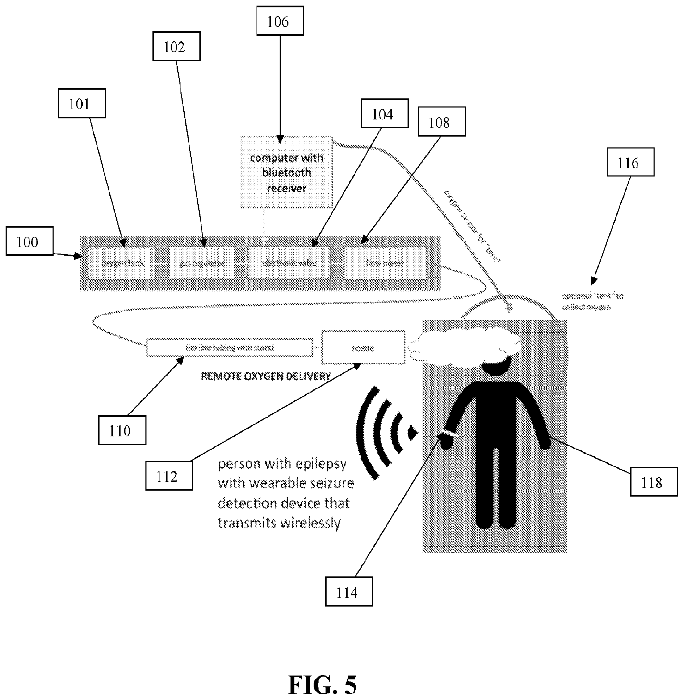

FIG. 5 depicts an embodiment of a system or device used for oxygen delivery and prevention of death due to seizure-induced laryngospasm or other transient acute upper airway obstruction. An oxygen tank with solenoid flow control and in line flow meter to set flow and remote nozzle can be positioned near a bedside. An optional "tent" or canopy can be placed to increase the concentration of atmospheric oxygen, especially in large rooms. The system or device may include the physical setup of the computer, oxygen tank, computer-controlled oxygen flow controls, tubing and stand to position the output nozzle close to the individual's head, and an optional canopy to help concentrate the oxygen around the individual's face. A wearable seizure detection device transmits to the computer, which activates a fixed duration stream of oxygen. The recommended oxygen stream duration is three minutes, but can be modified in the specific context by (1) additional seizure detection from the wearable and/or (2) an oxygen level detected by an oxygen sensor near the individual's head.

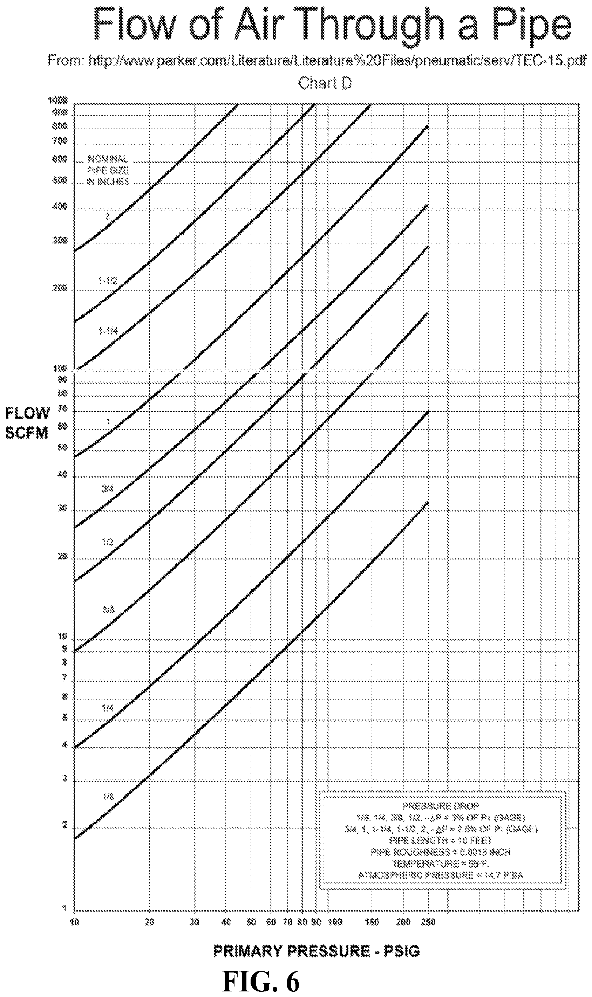

FIG. 6 shows the flow of air through a pipe in standard cubic feet per minute (SCFM). A flow of 100 scfm can be achieved with an oxygen source at 60 psi and a ¾ tubing over 10 feet. This flow for up to 3 minutes will provide sufficient oxygen to maintain the oxygen levels high enough to prolong the time to respiratory arrest.

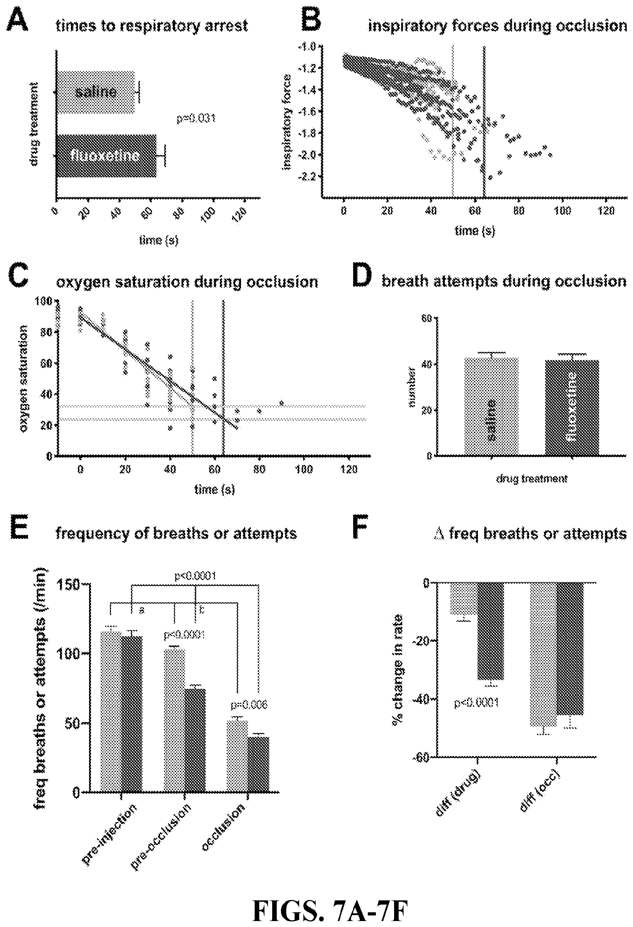

FIGS. 7A-7F show the impact of systemic fluoxetine (25 mg/kg IP) on time to respiratory arrest in a rat model of complete airway occlusion. In FIG. 7A, rats exposed to systemic fluoxetine experienced respiratory arrest at an average time after occlusion onset of 63.8 seconds. Saline injected control rats arrested at an average time of 49.8 seconds after occlusion onset (p=0.031). In FIG. 7B, there were no apparent differences in the inspiratory forces generated during breath attempts while occluded (the mean times to respiratory arrest are shown as vertical orange and blue lines). In FIG. 7C, the slopes of the linear regression lines did not differ statistically. The gray horizontal lines indicate the intersection of the linear regression line with the mean time to respiratory arrest. In FIG. 7D, there was no difference in the number of breath attempts between the two groups, but the increased time to respiratory arrest for the fluoxetine group indicated that the rate of breath attempts differed. In FIG. 7E, frequency of breath attempts pre-injection with fluoxetine or saline, post-injection (i.e. pre-occlusion), and during occlusion are compared. There was no difference between saline and fluoxetine groups in the pre-injection measures, but these groups differed from each other at later times (pre-occlusion p<0.0001, occlusion p=0.006). In one way ANOVA to compare pre-occlusion and occlusion to the pre-injection baseline condition for fluoxetine treated animals, both conditions differed from baseline (p<0.0001 after post hoc correction). Likewise, both pre-occlusion and occlusion differed from the pre-occlusion baseline for the saline treated animals (a, pre-occlusion p=0.013; b, occlusion p=0.0092; after post hoc correction). In FIG. 7F, the largest differential in respiratory (or respiratory attempts) rate was between baseline and drug or saline. Fluoxetine showed a much larger effect on the spontaneous breathing rate (p<0.0001). The differential rate between pre-occlusion and occlusion was comparable for fluoxetine and saline treated animals.

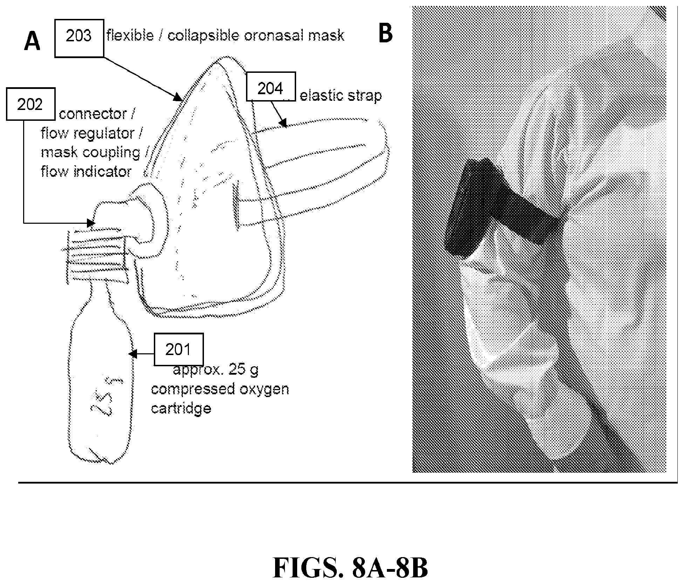

FIGS. 8A-8B depicts a portable system or device used for oxygen delivery and prevention of death due to seizure-induced laryngospasm or other transient acute upper airway obstruction. FIG. 8A shows the portable unit with major parts labeled. FIG. 8B shows a configuration of pouch and armband to make the unit accessible for wearer or bystander.

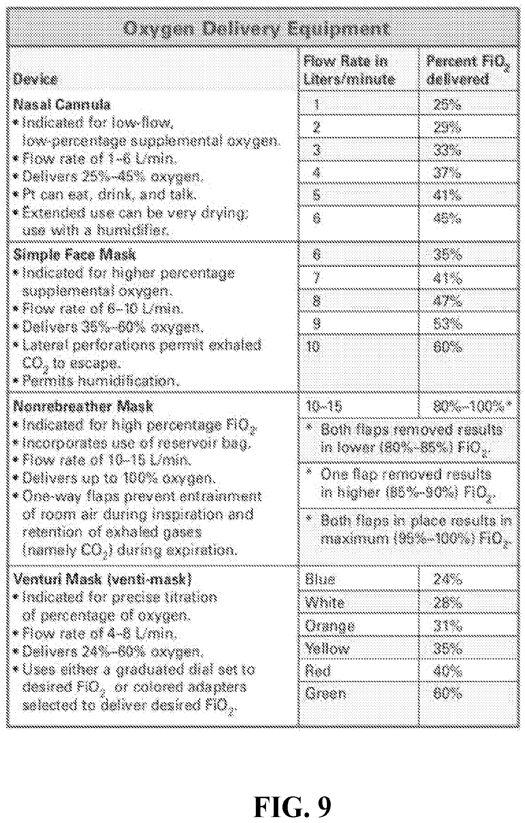

FIG. 9 shows a fraction of inspired oxygen (FiO2) associated with different flow rates of oxygen delivery equipment.

### DETAILED DESCRIPTION

A first aspect relates to a system for prevention of sudden death. The system includes a wearable seizure detection device; a base unit; and a computer, the computer configured to receive input from the seizure detection device and trigger the base unit to release oxygen under a condition effective to enrich an environmental oxygen level and prevent sudden death when the input indicates a seizure.

A second aspect relates to a method of preventing sudden death. The method includes enriching an environmental oxygen level for a sleeping subject where the subject has a seizure disorder using the system described herein.

It is to be appreciated that certain aspects, modes, embodiments, variations and features of the present disclosure are described below in various levels of detail in order to provide a substantial understanding of the present technology. The definitions of certain terms as used in this specification are provided below. Unless defined otherwise, all technical and scientific terms used herein generally have the same meaning as commonly understood by one of ordinary skill in the art to which this disclosure belongs.

As used herein, the term "about" means that the numerical value is approximate and small variations would not significantly affect the practice of the disclosed embodiments. Where a numerical limitation is used, unless indicated otherwise by the context, "about" means the numerical value can vary by ±1 or +10% , or any point therein, and remain within the scope of the disclosed embodiments.

Where a range of values is described, it should be understood that intervening values, unless the context clearly dictates otherwise, between the upper and lower limit of that range and any other stated or intervening value in other stated ranges, may be used in the embodiments described herein.

As used herein, the terms "subject," "individual" or "patient," used interchangeably, means any animal, including mammals, such as mice, rats, other rodents, rabbits, dogs, cats, swine, cattle, sheep, horses, or primates, such as humans.

It is further appreciated that certain features described herein, which are, for clarity, described in the context of separate embodiments, can also be provided in combination in a single embodiment. Conversely, various features which are, for brevity, described in the context of a single embodiment, can also be provided separately or in any suitable sub-combination.

The phrase seizure event as described herein includes, for example, a clinical episode where a subject or patient experiences any of various types of epileptic seizures, seizures associated with a seizure disorder, psychogenic or non-epileptic seizures (PNES), or other seizures.

The systems, devices, and methods described herein provide for the prevention of sudden death, particularly sudden death in epilepsy which manifests in the occurrence of seizure activity. Sudden death upon presence of a seizure (used interchangeably herein with sudden unexpected death in epilepsy (SUDEP)) generally occurs during or immediately after a seizure. Possible factors that may cause SUDEP include, for example, pauses in breathing that reduces the oxygen in the blood to a life-threatening level or obstruction to a person's airway. Other factors that may cause SUDEP include for example, a dangerous heart rhythm or heart failure, or SUDEP may result from more than one cause such as a combination of breathing difficulty and abnormal heart rhythm. Risk factors for SUDEP include, for example, uncontrolled or frequent seizures, generalized convulsive (e.g., tonic-clonic or grand mal) seizures, seizures that begin at a young age, a long-term history of epilepsy, lack of adherence to prescribed medicine, lack of proper sleep, and alcohol consumption.

The systems of the present disclosure provide for the protection of oxygen status during seizure activity. In accordance with the present disclosure, relatively brief access to an oxygen atmosphere just prior to airway occlusion leads to a significant prolonged the time to respiratory arrest, and, in some embodiments, may prevent the occurrence of respiratory arrest. In one embodiment of the present disclosure, as little as thirty seconds of oxygen immediately prior to airway closure can cause enough of an extension of the time to respiratory arrest that it would improve survival odds if seizure activity caused obstructive apnea. In another embodiment, thirty seconds would be within the time window of a seizure itself, suggesting that oxygen in accordance with the systems described herein is protective when supplied after a seizure starts.

Seizure-detection as described herein includes monitoring a patient for seizure-related activity. Seizure-detection may be run individually in a strategy for monitoring a patient or may be run in combination with other seizure-detection routines or methods in an overall strategy for patient monitoring. For example, a seizure detection device having a processor may execute a seizure-detection routine configured to process a signal in order to calculate one or more measurable properties of the signal and compare those to one or more thresholds in order to detect one or more seizure-related events.

Seizure-related muscle activity as described herein includes, for example, muscle activity that exhibits a measurable property that is increased or more prevalent during any of various types of epileptic seizures, seizures associated with a seizure disorder, psychogenic or non-epileptic seizures (PNES), or other seizures when compared to one or more levels of the property measured for a patient at rest and in a normal or non-seizure state. Properties that may be measured include, for example, those that increase or become prevalent during seizures such as muscle activity, coherence of muscle groups, levels of rhythmic or repetitive muscle activation, other properties associated with seizures, and combinations thereof. While some measurable properties may be more prevalent when a seizure is occurring, those properties may also be present or elevated, at least to some degree, during some non-seizure activities. Accordingly, measured or detected seizure-related activity may or may not be indicative of an actual seizure or epileptic seizure. The systems of the present disclosure may release oxygen upon detection of an actual seizure or epileptic seizure, or, alternatively, may release oxygen upon detection of an epileptic event that is non-seizure epileptic activity, without causing harmful or detrimental effects to the subject.

A seizure-related event as disclosed herein includes, for example, a clinical episode or event in which a subject exhibits seizure-related activity. A seizure-related event may or may not be associated with an actual seizure or epileptic seizure. In one embodiment, the system is used with an actual seizure, or alternatively, the system may be used with a seizure-related event that may or may not include an actual seizure or epileptic seizure.

The systems and methods described herein may be used upon detection and/or characterization of seizures or seizure-related events to automatically trigger the release of oxygen upon detection and/or characterization of seizures or seizure-related events. The systems and methods described herein may be configured for real-time release of oxygen and real-time monitoring of patients. The oxygen may be automatically released by the systems described herein upon detection of a seizure or seizure-related event. The systems and methods described herein may also be used to timely alert caregivers of seizure-related events and/or update an alert response. In one embodiment, the systems and methods described herein may be used to characterize or analyze historical or earlier collected data including, for example, patient data collected using one or more sensors which are described in detail *infra.*

The systems disclosed herein may include a wearable seizure detection device. The wearable seizure detection device may be used to verify presence of a seizure or seizure-related event and upon verification, the systems disclosed herein may automatically release oxygen, and may further be used to assist a caregiver in making a diagnosis. For example, in one embodiment, the systems and methods disclosed herein may be used to assist a caregiver in making a diagnosis that a patient is/has experienced a psychogenic or non-epileptic seizure (PNES) event or an epileptic event. The systems and methods described herein may include sensors having one or more electrodes disposed on, near, or underneath the skin of a patient and, in one embodiment, one or more electrodes may be implanted in a subject. Alternatively, one or more electrodes may be attached to a patient's clothing and may be configured for measurement of muscle electrical activity. In one embodiment, the systems and methods described herein may exclude, or exclude use of, a detection sensor, but may include, or include use of, one or more processors suitably configured to receive signal data or other sensor signal data and process the data to detect and/or characterize a seizure event or seizure-related event data. Detection of seizures is described in, for example, U.S. Pat. Nos. 8,983,591, 9,186,105, 9,439,595, and 9,439,596 to Leininger et al.

Seizure events or seizure-related events that may be detected using the seizure detection device described herein include generalized tonic-clonic seizure (GTC) events which may be caused by epilepsy. In one embodiment, the systems and methods disclosed herein may also be used to detect other type of seizure-related events including, for example, some that may result from conditions other than epilepsy. For example, some seizures that may share one or more characteristics with generalized tonic-clonic seizures commonly associated with epilepsy, such as increased muscle activity or increased repetitive muscle activity, may also be detected. In another example, in one embodiment, PNES events, which may be associated with conditions other than epilepsy, may be detected. Accordingly, PNES events may be detected and used to indicate that a patient may have a condition other than epilepsy. In one embodiment, such as where detection units may be disposed on more than one location or side of a patient's body, complex-partial seizures may also be detected. In one embodiment, the seizure disorder may be epilepsy or any neurological or other medical condition that is associated with generalized seizures, absence seizures, tonic-clonic seizures, focal seizures, simple focal seizures, complex focal seizures, secondary generalized seizures, or any other seizure disorder.

Treatment-resistant epilepsy (TRE) or intractable epilepsy, which may be treated in accordance with the systems and methods described herein, includes, for example, epilepsy that is not adequately controlled by trials of one or more anti-epileptic drugs (AED). In one embodiment, the systems and methods of the present disclosure may be used in the presence of TRE or intractable epilepsy.

Childhood epilepsy includes, for example, many different syndromes and genetic mutations that can occur to cause epilepsy in childhood. Examples include, but are not limited to, Tuberous Sclerosis Complex; Dravet Syndrome; Myoclonic-Absence Epilepsy; Lennox-Gastaut syndrome; Generalized Epilepsy of unknown origin; CDKL5 mutation; Aicardi syndrome; bilateral polymicrogyria; Dup15q; SNAP25; and febrile infection related epilepsy syndrome (FIRES); benign rolandic epilepsy; juvenile myoclonic epilepsy; infantile spasm (West syndrome); and Landau-Kleffner syndrome. The list above is non-exhaustive as many different childhood epilepsies exist. In one embodiment, the systems and methods of the present disclosure may be used in a subject having or at risk of having childhood epilepsy (e.g., a child).

Focal seizures as recited herein include, for example, seizures which originate within networks limited to only one hemisphere. What happens during the seizure depends on where in the brain the seizure happens and what that part of the brain normally does. In one embodiment, the systems and methods of the present disclosure may be used in a subject having or at risk of having focal seizures. Focal seizure where awareness/consciousness are impaired includes, for example, a seizure that usually starts in a small area of the temporal lobe or frontal lobe of the brain and involve other areas of the brain within the same hemisphere that affect alertness and awareness. Subjects may experience automatisms during a focal seizure with impaired consciousness.

Mixed seizures as recited herein include, for example, the existence of both generalized and focal seizures in the same subject. In one embodiment, the systems and methods of the present disclosure may be used in the presence of mixed seizures.

The systems described herein may include a base unit to house parts of the system. In one embodiment, the base unit comprises an oxygen tank, a gas regulator, an electronic valve, a flow meter, and a nozzle. In one exemplary embodiment, as depicted in FIG. 5, base unit 100 may house oxygen tank 101, gas regulator 102, electronic valve 104, and flow meter 108. A computer 106 may communicate and transmit signals to and from base unit 100, and may do so wirelessly, optionally via a Bluetooth or other wireless receiver and wireless connection, or via a wired connection. Base unit 100 may be attached to flexible tubing 110 with a stand which may be further attached to a nozzle 112. A subject 118 as described herein may wear a seizure detection device 114. The wearable seizure detection device 114 may optionally transmit the detection of a seizure or seizure-related event to the base unit 100 in a wireless or wired manner. In one embodiment, the at least one of the input and the trigger is transmitted wirelessly.

The signal may be transmitted via a hardware or software component configured to communicate data, such as measurements of vital signs, from the wearable seizure detection device to the base unit, for example. In one embodiment, the signal includes an active communication interface, for example, a modem, transceiver, transmitter-receiver, or the like. In one embodiment, the signal includes a passive communication interface, for example, a quick response (QR) code, Bluetooth identifier, radio-frequency identification (RFID) tag, a near-field communication (NFC) tag, or the like. The signal may operate over wired or wireless communication connections, such as, for example, a wireless network connection, a Bluetooth connection, an infrared connection, an NFC connection, a cellular network connection, a radio frequency connection, or any combination thereof. In one embodiment, the signal can use input and output, such as, for example, an ultrasonic signal or a sound signal via an audio jack. The signal may include a single component, one of each of the foregoing types of components, or any combination of the foregoing components.

FIG. 5 depicts an embodiment of a device used for oxygen delivery and prevention of death due to seizure-induced laryngospasm or other transient acute upper airway obstruction. The base unit 100 may include an oxygen tank 101 and a flow meter 108 which may set flow parameters. The system may further include a nozzle 112 that, in an example, may be remote and positioned near a bedside of subject 118. In one embodiment, the nozzle is attached to the base unit by a flexible tubing 110. In one embodiment, the flexible tubing has a length of more than about 1 foot. The length of the tubing may be lengthened or shortened based on the oxygen concentration, pressure, duration, and flow required for sufficient oxygen delivery to the subject based on the subject's condition and epileptic seizure or seizure-related event. For example, the flexible tubing may be more than about 2 feet, more than about 3 feet, more than about 4 feet, more than about 5 feet, more than about 10 feet, more than about 15 feet, more than about 20 feet, more than about 25 feet, more than about 30 feet, more than about 35 feet, more than about 40 feet, more than about 45 feet, more than about 50 feet, or any length of tubing therebetween. The length of the tube may be, for example, between about 1 to 100 feet, between about 25 and 75 feet, between about 30-70 feet, between about 40 to 60 feet, or may be about 1 foot, about 2 feet, about 3 feet, about 4 feet, about 5 feet, about 6 feet, about 7 feet, about 8 feet, about 9 feet, about 10 feet, about 15 feet, about 20 feet, about 25 feet, about 30 feet, about 35 feet, about 40 feet, about 50 feet, or any length therebetween.

In one embodiment, the base unit further includes an attachment device capable of providing a concentrated environmental oxygen level surrounding the attachment device. Such an attachment device may be in the shape of a tent, cone, or canopy in one embodiment. The optional tent or canopy 116 may be placed near the face or head area of subject 118 to increase the concentration of atmospheric oxygen in the area surrounding the head of the subject 118. This optional tent of canopy 116 is particularly useful in large rooms. In one embodiment, the canopy is a conical canopy. The optional tent or canopy 116 may collect oxygen and increase peripheral oxygen concentration levels around a subject's head. Features and details of the configuration of the hardware for oxygen release and delivery to a subject can be varied to accommodate different sleeping positions, tendencies to move during periods of sleep, bed structure, room size, and any other variable that could impact concentration of oxygen after release.

The system may include the physical setup of the computer 106, oxygen tank 101, flow meter 108, electronic valve 104, tubing 110 with a stand to position the output nozzle 112 close to the head of the subject, and an optional canopy or tent 116 to increase concentration of oxygen levels around the individual's face. A wearable seizure detection device 114 may transmit to the computer 106, which activates a fixed duration or continuous stream of oxygen. In one embodiment, the preferred oxygen stream duration is three minutes, but the duration, flow, and pressure of the oxygen stream can be modified in the specific context by additional seizure detection from the wearable detection device and/or based on an oxygen level detected by an oxygen sensor near the individual's head. In one embodiment, the base unit includes an oxygen sensor. The oxygen sensor may be used to provide feedback to computer 106 which can then trigger base unit 100 to release oxygen under a condition effective to enrich an environmental oxygen level and prevent sudden death when the input indicates a seizure.

The system may further include a different type of sensor (as shown in FIG.5), one that may measure the level of oxygen around the subject so that the flow of oxygen may be automatically adjusted. A commercially available oxygen sensor may be sufficient for monitoring local atmospheric oxygen level (i.e., the level of oxygen around a subject). The sensor may an include one or more sensors that can be disposed on any part of the wearable seizure detection device or any location near the subject's head. The sensor can be used to identify, detect, determine, or generate signal data indicative of measurements (e.g., electrical activity or motion) associated with the wearable seizure detection apparatus or an individual wearing the wearable seizure detection apparatus.

The sensor may include one or more sensors used to detect the state or condition of an environment, including electromyography sensors, accelerometers, gyroscopes, optical sensors, light emitters, microphones, and/or touch sensors. An accelerometer may include various axes including a three-axis, a six-axis, or a nine-axis accelerometer. Optical sensors may include red, green, blue (RGB) cameras, infrared cameras, monochromatic infrared cameras, or any other optical sensor that can capture still images or moving images. Light emitters may be infrared light emitting diodes (LED), infrared lasers, or any other suitable lights. The sensors may, for example, include one or more sensors that can generate heart activity signals such as an EEG sensor, a photoplethysmogram (PPG) sensor, an electromyogram (EMG) sensor, or the like.

In one embodiment, a sensor may include a first electrode arranged in an interior surface of the wearable seizure detection device, which can be positioned to be in contact with the skin of an individual when worn, and a second electrode arranged in an exterior surface of the device. The sensor may, for example, include sensors capable of generating biometric signals, such as ECG signals, through non-invasive techniques which do not penetrate or contact the skin of the individual.

The sensor may also include one or more bioimpedance sensors, microphones, temperature sensors, touch screens, finger readers, iris scanners, or a combination thereof. Implementations of the sensors may include a single sensor, one of each of the foregoing sensors, or any combination of the foregoing sensors. In one embodiment, the signal data can be identified, detected, determined, or otherwise generated based on any single sensor or combination of sensors included in the wearable seizure detection device.

In one embodiment, the system further includes a remote control, where the remote control is capable of providing on demand oxygen release, either by the subject or by a third party such as a caregiver, healthcare professional, emergency responder, guardian, or other caregiver.

A computer as described herein includes, for example, any programmable machine capable of executing machine-readable instructions. A computer may include but is not limited to a general-purpose computer, microprocessor, computer server, digital signal processor, or a combination thereof. A computer may include one or more processors, which may include part of a single machine or multiple machines. A computer program as used herein includes a list of instructions that may be executed by a computer to cause the computer to operate in a desired manner. A computer readable medium as recited herein includes any tangible article of manufacture having a capacity for storing one or more computer programs, one or more pieces of data, or a combination thereof. A computer readable medium may include but is not limited to a computer memory, hard disk, memory stick, magnetic tape, floppy disk, optical disk (such as a CD or DVD), flash drive, zip drive, or combination thereof. In one embodiment, the computer disclosed herein is configured to receive input from the seizure detection device and then trigger the base unit to release oxygen under a condition effective to enrich an environmental oxygen level and prevent sudden death when the input indicates a seizure. Although the examples herein can be practiced with a single processor as shown, advantages in speed and efficiency can be achieved using more than one processor.

Detection as used herein includes, for example, any means of identifying the presence of something. For example, detection of a seizure-related event may refer to the identification of a seizure-related event in a portion of a collected input or signal. Where reference is made to detection of a seizure-related event, such detection may refer to the identification of a seizure-related event in one or more collected input signals. Detection of seizure-related events may include use of signals collected and processed in real-time, signals analyzed following collection in a post-process routine, or both.

The computer as described herein may be configured to receive input from the seizure detection device and trigger the base unit to release oxygen under a condition effective to enrich an environmental oxygen level and prevent sudden death when the input indicates a seizure or presence of a seizure-related activity. In one embodiment, input of seizure or seizure-related activity may be processed by a computer and used to determine values of one or more characteristics of seizure events or seizure-related events, which may, for example, be included in a quantitative summary of characteristics of event activity. That information may then be provided to a computer to trigger the release of oxygen and it may optionally be provided to one or more caregivers. For example, a quantitative summary of characteristics of a detected seizure may be created and may include, by way of nonlimiting example, the duration of phases or parts of the seizure, including the tonic phase, clonic phase, entire seizure, and any combinations thereof. In one embodiment, the intensity of one or more phases of a seizure or of an entire seizure may also be determined and may trigger the release of oxygen and further be included in a summary provided to one or more caregivers. In one embodiment, other characteristics associated with seizure events or seizure-related events, including, for example, characteristics of samples including elevated amplitude qualified as being related to clonic-phase activity, may also be determined, and used by the system to determine whether and when to release oxygen to enrich an environmental oxygen level. For example, the systems and methods described herein, whether explicitly may sometimes be executed together with other methods which may be suitable to trigger the release of oxygen and optionally provide a statistical summary of qualified samples to one or more caregivers. For example, in one embodiment, transition times into and/or out of one or more phases of a seizure may be determined using methods herein, and those transition times may be used to organize or verify data, for example, to link burst data to the clonic phase of a seizure, so that appropriate data is properly linked to the clonic phase and may be more accurately characterized in some other way. *See* for example, U.S. Patent Publ. No. 2017/0296083 to Cardenas et al.

In some embodiments, data (e.g., seizure-related event data and/or other data) may be collected and may further be identified, selected, and/or removed from other data. Data recording may be included as searchable meta-data included along with other signal data in a medical database. The systems described herein may monitor a subject, for example, while resting, such as during evening and nighttime hours.

Data may be collected and stored in the systems of the present disclosure. Collected data may be raw data, data collected in real-time, or data in some other form, such as without prior detection or marking of seizure or seizure-related events. In one embodiment, the system may be configured to analyze raw data or other data in order to detect seizure-related events that may not have been previously identified or marked. For example, data may be processed using one or more seizure-detection routines configured to detect one or more seizures or seizure-related events and select or remove one or more portions of data including the one or more seizure-related events.

In one embodiment, data may be transmitted from one medical database to one or more processors having instructions for executing one or more additional steps, such as, for example, further administration of oxygen. In one embodiment, a computer processor may include one or more medical databases and may also include one or more installed programs suitable for execution of the release of oxygen. In one embodiment, a personal or mobile detection unit may include a computer processor configured to execute the release of oxygen.

In one embodiment, detection of a seizure-related event may indicate a high confidence that a true seizure (e.g., a generalized tonic-clonic seizure or other seizure type commonly associated with epilepsy and/or the seizure disorder) may be occurring or may have occurred. In one embodiment, selection of data may include detection of one or more seizure-related events which, while showing signs of being associated with a true seizure, may or may not indicate the presence of a true seizure or that a seizure was detected at high confidence. For example, in one embodiment, data may include detection of one or more signal amplitudes or detection of a rate of change of signal amplitude that may be elevated above some suitable threshold level. Those amplitudes and/or rates of signal amplitude change may indicate an increased risk of seizure occurrence, but those conditions may be insufficient to fully discriminate signals from some non-seizure sources which may also produce elevated signal data. In one embodiment, additional steps may be used to classify a detected seizure-related event and increase confidence that selected data may likely be associated with a true seizure. Alternatively, additional steps may be used to identify that a detected seizure-related event may properly be categorized as a non-seizure movement or other seizure-related event type other than those of a true seizure. Thus, in one embodiment, processing of collected signal may create low possibility of false positive seizure detection and, thus, low pre-emptive administration of oxygen when unnecessary. In another embodiment, oxygen is administered upon detection of a signal indicating seizure occurrence or any seizure-related occurrence, without any harmful effect.

The systems described herein may automatically release oxygen under a condition effective to enrich an environmental oxygen level and prevent sudden death when input from a seizure detection device indicates presence of a seizure (or, alternatively, a seizure-related event). In one embodiment, oxygen is released within about 30 seconds after a seizure is detected by the seizure detection device. The systems disclosed herein may prolong the time to respiratory arrest in a subject having a seizure or seizure-related event.

The systems and methods disclosed herein substantially enrich the environmental oxygen level beyond ambient oxygen levels. For example, environmental oxygen level may be enriched by between about 1% and about 100% of the ambient oxygen levels. In particular, the environmental oxygen level may be enriched by about 1%, about 5%, about 10%, about 15%, about 20%, about 25%, about 30%, about 35%, about 40%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, or any amount therebetween. The environmental oxygen level may be enriched by, for example, between about 25% and 100%, between about 30% and 100%, between about 35% and 100%, between about 40% and 100%, between about 45% and 100%, between about 50% and 100%, between about 55% and 100%, between about 60% and 100%, between about 65% and 100%, between about 70% and 100%, between about 75% and 100%, between about 80% and 100%, between about 85% and 100%, between about 90% and 100%, between about 95% and 100%, between about 25% and 75%, between about 30% and 65%, between about 35% and 60%, between about 40% and 60%, between about 40% and 55%, between about 45% and 55%, between about 45% and 50%, or any combination of ranges therebetween. Alternatively, the environmental oxygen level may be enriched, for example, by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or any amount therebetween. In one embodiment, the condition effective to enrich an environmental level of oxygen is effective to enrich an environmental oxygen level to at least 50%.

The oxygen release by the systems and methods of the present disclosure may be completed under, in one embodiment, sustained release conditions. Sustained release conditions as described herein include a continuous release of oxygen for period of time, without interruption or pause of oxygen release. In one embodiment, the continuous release of oxygen occurs until a caretaker, caregiver, or healthcare provider stops the oxygen release, either manually or by control remotely. Alternatively, the oxygen release under sustained conditions may continue until a subject experiencing a seizure or seizure-related event stops the flow of oxygen. The oxygen may be released continuously or in intervals for an indeterminate period of time. For example, the oxygen may be release for at least 30 about seconds, at least about 1 minute, at least about 2 minutes, at least about 3 minutes, at least about 4 minutes, at least about 5 minutes, or any amount therebetween, or longer. If administered in intervals, the oxygen may be released for a set period of time (release) followed by a set period of time with no oxygen release (no release), followed by repeating the release and no release intervals for a sustained period of time effective to treat a seizure or seizure-related event and/or prevent sudden death. For example, oxygen may be released for intervals lasting less than 1 second, at least 1 second, at least 5 seconds, at least 10 seconds, at least 20 seconds, at least 30 seconds, or any amount of time deemed useful in preventing sudden death and/or protecting oxygen status during a seizure or seizure-related event. The non-release intervals may last, for example, less than 1 second, at least 1 second, at least 5 seconds, at least 10 seconds, at least 20 seconds, at least 30 seconds, or any amount of time deemed useful in preventing sudden death and/or protecting oxygen status during a seizure or seizure-related event.

In another embodiment, the oxygen release may be for a fixed duration of time. A fixed duration of time as described herein may include release of oxygen for a predetermined duration of time effective treat a seizure or seizure-related event and/or prevent sudden death. In a fixed duration release, the systems may be programed to release oxygen continuously or in intervals for a set duration of time and may automatically cease administration of oxygen release at the end of the set duration of time. For example, the oxygen may be released for a fixed duration of time including, but not limited to, between about 5 seconds and about 15 minutes, between about 30 seconds and about 15 minutes, between about 1 minute and about 15 minutes, between about 2 minutes and about 15 minutes, between about 3 minutes and about 15 minutes, between about 4 minutes and about 15 minutes, between about 5 minutes and about 15 minutes, between about 30 seconds and about 10 minutes, between about 1 minute and about 10 minutes, between about 2 minutes and about 10 minutes, between about 3 minutes and about 10 minutes, between about 4 minutes and about 10 minutes, between about 5 minutes and about 10 minutes, between about 30 seconds and about 5 minutes, between about 1 minute and about 5 minutes, between about 2 minutes and about 5 minutes, between about 3 minutes and about 5 minutes, between about 4 minutes and about 5 minutes, or any amount of time therebetween. In one embodiment, the fixed duration of time is for a period of between about 30 seconds to about 5 minutes. The fixed duration of time release of oxygen may also be administered under interval conditions as described *supra.*

The pressure and flow of oxygen release may vary and be dependent on the length of tubing and diameter of tubing used in the system. In one embodiment, a flow of 100 standard cubic feet per minute (SCFM) can be achieved with an oxygen source at 60 psi and a ¾ tubing over 10 feet. In such an embodiment, this flow for up to 3 minutes may provide sufficient oxygen to maintain the oxygen levels high enough to prolong the time to respiratory arrest.

The oxygen may be released in any flow useful to treat or mitigate a seizure or seizure-related event. For example, the oxygen may be released at a flow rate of at least 1 standard cubic feet per minute. The oxygen may be released at a flow rate of, for example, at least 2.5 standard cubic feet per minute, at least 5 standard cubic feet per minute, at least 10 standard cubic feet per minute, at least 15 standard cubic feet per minute, at least 20 standard cubic feet per minute, at least 25 standard cubic feet per minute, at least 30 standard cubic feet per minute, at least 35 standard cubic feet per minute, at least 40 standard cubic feet per minute, at least 45 standard cubic feet per minute, at least 50 standard cubic feet per minute, at least 55 standard cubic feet per minute, at least 60 standard cubic feet per minute, at least 65 standard cubic feet per minute, at least 70 standard cubic feet per minute, at least 75 standard cubic feet per minute, at least 80 standard cubic feet per minute, at least 85 standard cubic feet per minute, at least 90 standard cubic feet per minute, at least 95 standard cubic feet per minute, at least 100 standard cubic feet per minute, at least 105 standard cubic feet per minute, at least 110 standard cubic feet per minute, at least 115 standard cubic feet per minute, at least 120 standard cubic feet per minute, at least 125 standard cubic feet per minute, at least 130 standard cubic feet per minute, at least 135 standard cubic feet per minute, at least 140 standard cubic feet per minute, at least 145 standard cubic feet per minute, at least 150 standard cubic feet per minute, at least 155 standard cubic feet per minute, at least 160 standard cubic feet per minute, at least 165 standard cubic feet per minute, at least 170 standard cubic feet per minute, at least 175 standard cubic feet per minute, at least 180 standard cubic feet per minute, at least 185 standard cubic feet per minute, at least 190 standard cubic feet per minute, at least 195 standard cubic feet per minute, at least 200 standard cubic feet per minute, at least 205 standard cubic feet per minute, at least 210 standard cubic feet per minute, at least 215 standard cubic feet per minute, at least 220 standard cubic feet per minute, at least 225 standard cubic feet per minute, at least 230 standard cubic feet per minute, at least 235 standard cubic feet per minute, at least 240 standard cubic feet per minute, at least 245 standard cubic feet per minute, at least 250 standard cubic feet per minute, or any flow rate therebetween. In one embodiment, oxygen is released at a flow of between about 1 and about 250 standard cubic feet per minute.

The oxygen may be released in any pressure useful to treat or mitigate a seizure or seizure-related event. For example, the oxygen may be released at a pressure of at least 1 psi. The oxygen may be released at a pressure of, for example, at least 2 psi, at least 3 psi, at least 4 psi at least 5 psi, at least 6 psi, at least 7 psi, at least 8 psi, at least 9 psi, at least 10 psi, at least 15 psi, at least 20 psi, at least 25 psi, at least 30 psi, at least 35 psi, at least 40 psi, at least 45 psi, at least 50 psi, at least 55 psi, at least 60 psi, at least 65 psi, at least 70 psi, at least 75 psi, at least 80 psi, at least 85 psi, at least 90 psi, at least 95 psi, at least 100 psi, or any amount therebetween. The pressure of the oxygen release may, for example, be between 1 psi and 100 psi, between 10 psi and 100 psi, between 20 psi and 100 psi, between 30 psi and 100 psi, between 40 psi and 100 psi, between 50 psi and 100 psi, between 60 psi and 100 psi, between 70 psi and 100 psi, between 80 psi and 100 psi, between 90 psi and 100 psi, and any range therebetween. In one embodiment, the oxygen is released at a pressure of between about 1 psi and about 100 psi.

A third aspect relates to a portable system for prevention of sudden death. The portable system includes an oronasal mask; a compressed oxygen cartridge, wherein the compressed oxygen cartridge is coupled to the oronasal mask; and a fastener, wherein the fastener is coupled to the oronasal mask, wherein the system releases oxygen upon activation under a condition effective to enrich an environmental oxygen level and prevent sudden death.

This aspect is carried out in accordance with the previously described aspects.

The oronasal mask may, for example, be made of any material that provides sufficient flexibility to accommodate a user's face, in particular, a user's mouth and nose area. In one embodiment, the oronasal mask is flexible. The mask may be made of various types of silicone, plastic, and/or polymers that allow for sufficient flexibility and performance on a user, for example, polyethylene. The mask may contain one or more additives in the silicone, plastic, and/or polymer such as, for example, anti-static additives, ultraviolet stabilizers, color or other pigment, flame or fire retardant, slip or anti-block agents, external coatings, and any combination of these additives. In one embodiment, the oronasal mask is collapsible.

The portable system may include a fastener that may be coupled to the oronasal mask. The fastener may be of any material or physical embodiment that allows for convenient positioning of the oronasal mask to a user's oral-nasal area. In one embodiment, the fastener is an elastic strap.

The portable system may further include a compressed oxygen cartridge, and the compressed oxygen cartridge may be coupled to the oronasal mask. The portable system may release oxygen upon activation under a condition effective to enrich an environmental oxygen level and prevent sudden death in a user and, in one embodiment, may do so automatically upon detection of a seizure event.

The pressure and flow of oxygen release may vary and be dependent on the compressed oxygen cartridge and other factors such as positioning and placement of the oronasal mask on a user's oronasal area when the system is in use. The pressure and flow of oxygen, in various embodiments, may be carried out in accordance with previously described aspects of the disclosure.

The compressed oxygen cartridge may, for example, be between about 1-gram and about 50-grams. For example, the compressed oxygen cartridge may be about 1-gram, about 5-grams, about 10-grams, about 15-grams, about 20-grams, about 25-grams, about 30-grams, about 35-grams, about 40-grams, about 45-grams, about 50-grams, and any amount therebetween. The compressed oxygen cartridge may be less than about 1-gram or more than about 50-grams. In one embodiment, the compressed oxygen cartridge is at least about 5-grams. The portable system may include a single compressed oxygen cartridge or may include more than one compressed oxygen cartridge. In one embodiment, the system includes one or more additional compressed oxygen cartridges. For example, the system may include one compressed oxygen cartridge, two compressed oxygen cartridges, three compressed oxygen cartridges, four compressed oxygen cartridges, five compressed oxygen cartridges, or more than five compressed oxygen cartridges.

The portable system and method disclosed herein substantially enrich the environmental oxygen level beyond ambient oxygen levels in accordance with the previously described aspects of the disclosure. For example, environmental oxygen level may be enriched by between about 1% and about 100% of the ambient oxygen levels. In particular, the environmental oxygen level may be enriched by about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or more than about 90%. In one embodiment, the condition is effective to enrich an environmental oxygen level to at least 40%.

In one embodiment, the oxygen cartridge is coupled to the oronasal mask by a flexible tubing. In one embodiment, the flexible tubing has a length of less than about 10 feet. In such an embodiment, a particular flow may provide sufficient oxygen to maintain the oxygen levels high enough to prolong the time to respiratory arrest. The oxygen may be released at a flow of between about 1 liter per minute and about 50 liters per minute. The oxygen may be released at a flow of about 1 liter per minute, about 2 liters per minute, about 3 liters per minute, about 4 liters per minute, about 5 liters per minute, about 10 liters per minute, about 15 liters per minute, about 20 liters per minute, about 25 liters per minute, about 30 liters per minute, about 35 liters per minute, about 40 liters per minute, about 45 liters per minute, about 50 liters per minute, and any amount therebetween. In one embodiment, oxygen is released at a flow of between about 1 and about 25 liters per minute.

The pressure of the oxygen released may be carried out in accordance with previously described aspects, and, in one embodiment, may be between about 1 psi and about 100 psi. The system, in one embodiment, may further include a flow indicator which is capable of detecting oxygen flow. In one embodiment, the system further includes a flow regulator which is capable of regulating or controlling oxygen flow. The system, in one embodiment, may further include an oxygen sensor as described herein.

In another embodiment, the system further includes a storage pouch, which may be useful for storing the portable system and further to maintain cleanliness and sanitation of the portable system. In yet another embodiment, the system further includes an armband, for example an elastic band, which may be useful for storing the portable system in a convenient location to a user's nose and mouth area, for example, on a user's arm.

In one embodiment, the portable system includes a wearable seizure detection device in accordance with the embodiments disclosed *supra.* In one embodiment, the portable system may automatically administer oxygen by triggering the release of oxygen upon detection and/or characterization of seizures or seizure-related events in accordance with the embodiments disclosed *supra.*

FIGS. 8A-8B depict embodiments of a portable device used for oxygen delivery and prevention of death due to seizure-induced laryngospasm or other transient acute upper airway obstruction as described herein. The portable system may be carried conveniently in a compact armband pouch as shown in FIG. 8B and may include elements in addition to the pouch and armband. For example, the portable system may include a cartridge filled with compressed oxygen (201), for example a cartridge that contains approximately 25-grams of compressed oxygen. The portable system may also include a connector (202) to which the oxygen cartridge is connected, punctured for the controlled release of gas, a fixed flow regulator with various flow options (for example, in one embodiment, 8 liter/min and/or 4 liter/min flow options), an indicator of flow or no flow, and attachment to a mask. Further, the portable system may include a soft, flexible, collapsible oronasal mask (203) (made from, for example, silicone) that has holes for a strap and an elastic strap that will go around the back of the head and hold the mask over the mouth and nose (204). When in operation, the portable device may, for example, be activated and placed over the mouth and nose by an individual who believes that a seizure is about to occur or an individual sees a seizure beginning. This can be based on the experience of aura, which many seizure patients report, other sense that a seizure is about to happen, the report of a seizure prediction device, and/or by alert of a seizure detection device. In this mode, the individual may activate the device (or the device may automatically activate) and secure the strap to deliver oxygen even if the individual loses consciousness (which happens in generalized seizures).

A fourth aspect relates to a method of preventing sudden death, the method including enriching an environmental oxygen level for a subject using the portable system for prevention of sudden death described herein, wherein the subject has a seizure disorder.

This aspect is carried out in accordance with the previously described aspects.

For purposes of this and other aspects of the disclosure, the target "subject" encompasses any animal, preferably a mammal, more preferably a human. In the context of administering a composition of the disclosure for purposes of preventing prevention of sudden death in a subject, the target subject encompasses any subject that is at risk of experiencing a seizure and or being diagnosed with epileptic seizure. Particularly susceptible subjects include adults and elderly adults. However, any infant, juvenile, adult, or elderly adult at risk for epileptic seizure can be treated in accordance with the methods of the present disclosure.

As used herein, the phrase "therapeutically effective amount" means an amount of oxygen that elicits the biological or medicinal response that is being sought in a tissue, system, animal, individual or human by a researcher, veterinarian, medical doctor or other clinician. As such, the therapeutic effect can be a decrease in the severity of symptoms associated with the disorder and/or inhibition (partial or complete) of progression of the disorder, or improved treatment, healing, prevention or elimination of a disorder, or side-effects. The amount needed to elicit the therapeutic response can be determined based on the age, health, size, and sex of the subject. Optimal amounts can also be determined based on monitoring of the subject's response to treatment. The term "treatment" or "treat" may include effective inhibition, suppression or cessation of the epileptic activity so as to prevent or delay the onset, retard the progression, or ameliorate the symptoms of the epileptic or seizure-related event.

As used herein, the term "simultaneous" therapeutic use refers to the administration of at least one additional agent beyond the oxygen, for example, agents administered before, during, or after an epileptic event or seizure, optionally, by the same route and at the same time or at substantially the same time. As used herein, the term "separate" therapeutic use refers to an administration of at least one additional agent beyond the oxygen, for example, agents administered before, during, or after an epileptic event or seizure, at the same time or at substantially the same time by different routes. As used herein, the term "sequential" therapeutic use refers to administration of at least one additional agent beyond the oxygen, for example, agents administered before, during, or after an epileptic event or seizure, at different times, the administration route being identical or different. More particularly, sequential use refers to the whole administration of the additional agent before administration of the oxygen. It is thus possible to administer the additional agent over several minutes, hours, or days before applying the oxygen. In one embodiment, the additional agent is administered before, during, or after the epileptic event or seizure.

In the following description, reference is made to the accompanying drawings that form a part hereof, and in which is shown by way of illustration specific embodiments which may be practiced. These embodiments are described in detail to enable those skilled in the art to practice the technology, and it is to be understood that other embodiments may be utilized and that structural, logical and electrical changes may be made without departing from the scope of the present technology, The following description of example embodiments is, therefore, not to be taken in a limited sense.

The present disclosure may be further illustrated by reference to the following examples.

### EXAMPLES

The following examples are intended to illustrate, but by no means are intended to limit, the scope of the present disclosure as set forth in the appended claims.

### Example 1 - Oxygen Prolongs the Time to Respiratory Arrest and Protects Against Death.

The present disclosure shows that the mechanism for protection against death by oxygen is a prolongation of the time to respiratory arrest (FIGS. 1A-1F and 2A-2B). Exposure to oxygen before the onset of obstructive apnea delays the time to respiratory arrest significantly but does not prolong seizure activity (FIG. 3).

The devices and systems to prevent death as disclosed herein may use a seizure detection algorithm or any other available seizure detection device (e.g., the Embrace2 wearable seizure detection device by Empatica) to trigger a stream of oxygen onto an individual in their bed (*see* FIG. 4). The delivery of oxygen, triggered approximately 30 seconds or more prior to complete airway occlusion due to laryngospasm or other cause, will delay any potential respiratory arrest and protect the individual from death due to laryngospasm-based airway obstruction. By preventing respiratory arrest, the device protects individuals who may not have a caregiver available for resuscitation if they experienced respiratory arrest.

The details of the configuration of the hardware for stream delivery can be varied to accommodate different sleeping positions, tendencies to move during periods of sleep, bed structure, etc. (*see* FIG. 4). The fundamental variable is pushing sufficient oxygen into the environment around the individual's head to get the oxygen concentration locally as high as possible. The total time for the oxygen stream is typically ≤3 minutes because the average duration of a tonic-clonic seizure is 1 to 3 minutes (*see* "Types of Seizures," Epilepsy Foundation (2019).

An example embodiment of the device disclosed herein which is capable of delivering a cloud of oxygen to prevent death due to seizure-induced laryngospasm or other transient acute upper airway obstruction is shown in FIG. 5. An oxygen tank 100 with solenoid flow control and in line flow meter to set flow and remote nozzle can be positioned near bedside. An optional "tent" or canopy 116 can be placed to increase the concentration of atmospheric oxygen, especially in large rooms. An optional oxygen sensor may be positioned near the individual that can be used to feed back to the computer to sustain flow. In one configuration, operation of the device consists of the physical setup of the computer 106, oxygen tank 101, computer controlled oxygen flow controls, tubing and stand 110 to position the output nozzle close to the individual's head, and an optional canopy to help concentrate the oxygen around the individual's face. A wearable seizure detection device 114 transmits via Bluetooth to the computer 106, which activates a fixed duration stream of oxygen. The recommended oxygen stream duration is three minutes but can be modified in the specific context by (1) additional seizure detection from the wearable and/or (2) an oxygen level detected by an oxygen sensor near the individual's head.

Depending upon the size of the room in which it is used, at 100 scfm, it is expected that atmospheric oxygen concentrations are ≥50% during most of a three-minute duration stream. With the optional "tent" or canopy, it is expected that the same oxygen stream to be able to significantly exceed 80% oxygen in the atmosphere around the face of the individual.

### Example 2 - Materials and Methods.

Methods and implants were similar to those described in additional detail previously (*see, e.g.,* Mooney et al., "Diving Responses Elicited by Nasopharyngeal Irrigation Mimic Seizure-Associated Central Apneic Episodes in a Rat Model," Neurobiol. Dis. 124:408-415 (2019); Nakase et al., "Laryngospasm, Central and Obstructive Apnea During Seizures: Defining Pathophysiology For Sudden Death in a Rat Model," Epilepsy Res. 128:126-139 (2016); Villiere et al., " A Resuscitation Option for Upper Airway Occlusion Based on Bolus Transtracheal Lung Inflation," Laryngoscope Investig. Otolaryngol. 3:296-303 (2018). All procedures were approved by an Animal Care and Use Committee and conducted in accordance with the United States Public Health Service's Policy on Humane Care and Use of Laboratory Animals. Adult male Sprague Dawley rats (AGE median=63, mean=88 days; WEIGHT median=306, mean=329 grams; Envigo Chicago, IL) were housed in AAALAC-accredited facilities and maintained on a 12 hour light:dark cycle with a temperature of 23°C and humidity of 55%, monitored daily, and had unrestricted access to water and food. Urethane (1.5 g/kg ip) was used for anesthesia in all animals. Euthanasia at the end of experiments included overdosing animals with Euthasol (150 mg/kg).

*ECG RECORDINGS -* Limb-lead ECG was recorded using copper strips coated with conductive gel wrapped around both forelimbs and the base of the tail. Signals were amplified and filtered to pass 1 Hz to 1 kHz (Model 1800 AC amplifier with headstages; A-M Systems, Sequim, WA) and digitized at 2 kHz (Micro1401 controlled by Spike 2 software; Cambridge Electronic Design, Cambridge, UK). Rhythm was assessed by reviewing P waves and associated QRS complexes for variations in wave shape, beat-to-beat intervals, and atrial- ventricular coupling.

*PULSE OXIMETRY -* Arterial oxygen saturation was measured by using a clip sensor on the thigh (TDR-43C, Med Associates, St Albans, VT) coupled to a pulse oximeter (CANL- 425SV-A, Med Assoc.). The raw pulsatile waveform of the pulse oximeter was digitized (2 kHz) with other signals.

*INSPIRATORY AIRWAY PRESSURES -* A pressure transducer (CyQ, Columbus Instruments, Columbus, OH) was connected to the sidearm of a T-shaped tracheal tube *(see infra).* Inspiratory pressures during complete airway occlusion were measured relative to those observed during baseline breathing. Signals were digitized along with other signals recorded at the same time.

*TRACHEAL IMPLANTS -* A T-shaped tracheal tube was placed through an incision between cartilaginous rings of the trachea after the trachea was exposed in the neck. The distal arm was "sealed" in the trachea with suture around the trachea, compressing it against the outside wall of the implant.

*CONTROLLED AIRWAY OCCLUSION -* Obstructive apnea was produced with controlled airway occlusion. The exterior arm in the straight path with the implanted arm served breathing or could be occluded. A pressure transducer on the tracheal tube sidearm recorded forces developed during either normal breathing with the tracheal tube open to the atmosphere or during complete closure of the open port. It has been shown that during occlusion, respiratory effort exerted to inspire progressively increased with each attempt until effort ceased and this point of respiratory arrest was associated with acute left ventricular dilatation, hypokinesis, and asystole (Nakase et al., "Laryngospasm, Central and Obstructive Apnea During Seizures: Defining Pathophysiology For Sudden Death in a Rat Model," Epilepsy Res. 128:126-139 (2016). To control the atmosphere available to the animal, the open port was connected to a stream of air, oxygen, or air/oxygen mixture that flowed continuously past the open port and could be switched to control the amount of time that the animal had access to a particular gas.

*SSRI PRETREATMENT* - Animals were injected intraperitoneally with fluoxetine (25 mg/kg made as a 2 mg/ml solution in saline to ensure solubilization of drug) or with an equivalent volume of saline. Injected volumes were 12.5 ml/kg and averaged 4.25 ± 1.00 ml/animal (mean, standard deviation). Fluoxetine hydrochloride (cat# 1279804) was obtained from Sigma Aldrich Inc. (St. Louis, MO).

*OXYGEN-RICH ATMOSPHERE EXPOSURE* - Animals were randomly assigned to one of 4 groups: (1) exposure to an oxygen atmosphere for 5 minutes prior to airway occlusion, (2) exposure to an air stream for 5 minutes prior to airway occlusion, (3) switching to an oxygen stream for only 30 seconds prior to airway occlusion, or (4) switching to a mixed stream (50% oxygen/50% air) for only 30 seconds prior to airway occlusion. Gas was delivered at ~4 liters/min. In all animals, the occlusion was left in place until 10 seconds after the point of respiratory arrest, which was defined as the time of the peak of the last breath attempt. Each animal was resuscitated as described previously (Villiere et al., " A Resuscitation Option for Upper Airway Occlusion Based on Bolus Transtracheal Lung Inflation," Laryngoscope Investig. Otolaryngol. 3:296-303 (2018), but only the first occlusion event in each animal was analyzed in this study (repeated occlusions change the response profile to occlusion as described in (Mooney et al., "Diving Responses Elicited by Nasopharyngeal Irrigation Mimic Seizure-Associated Central Apneic Episodes in a Rat Model," Neurobiol. Dis. 124:408-415 (2019). Planned group sizes were 8 animals per group. All groups have ≥8 per group except the 50% oxygen group (n=7) because one animal from this group was excluded due to improper gas mixing.

*SSRI PRETREATMENT -* Animals were randomly assigned to one of 2 groups: (1) systemic injection of fluoxetine (25 mg/kg IP) sixty minutes prior to occlusion testing, or (2) intraperitoneal saline of an equivalent volume based on animal weight 60 min prior to occlusion testing (*e.g.*, Tupal et al., "Evidence Supporting a Role of Serotonin in Modulation of Sudden Death Induced by Seizures in DBA/2 Mice," Epilepsia 47:21-6 (2006) and Zeng et al., "Fluoxetine Prevents Respiratory Arrest Without Enhancing Ventilation in DBA/1 Mice," Epilepsy Behav. 45:1-7 (2015). Occlusion testing was identical to that described for the oxygen exposed animals. Group sizes were 8 animals per group.

*Data analysis and statistics -* Analyses were done with Spike 2 software (Cambridge Electronic Design, Ltd., Cambridge, England) and Microsoft Excel. Data are reported as means ± standard deviation or standard error of the mean in the text and figures. All statistics were computed with GraphPad Prism 8 software or IBM SPSS Statistics (version 24). A p < 0.05 was predefined, after appropriate post-hoc correction for multiple comparisons where appropriate, to be statistically significant.

Fifty-four adult male animals were used. Thirty-seven was the final number of animals in the oxygen atmosphere series and 16 animals were used in the SSRI series.

### Example 3 - Exposure to an Oxygen-Rich Atmosphere Prior to Airway Occlusion Prolonged the Time to Respiratory Arrest.

Exposure to an oxygen-rich atmosphere prior to airway occlusion prolonged the time to respiratory arrest. Examples of individual experiments on animals exposed to either an air stream or an oxygen stream for five minutes prior to airway occlusion are shown in FIGS. 2A-2B and multiple measures are summarized in FIGS. 1A-1F. Key features that differentiate the responses are (1) a significantly longer time to respiratory arrest in animals exposed to oxygen, (2) ECG changes indicative of hypoxia (ST segment elevation, rate decreases due to missed QRS complexes, premature ventricular beats) preceding the point of respiratory arrest, (3) a pattern of inspiratory attempts that rapidly increases as the animal approaches the point of respiratory arrest, and (4) minimum pulse oximetry levels that were comparable across all groups (37% ± 11 for oxygen animals, n=12, 30% ± 9 for air animals, n=8; mean, standard deviation).

Interestingly, the breath attempts made during the first half of the occlusion period during oxygen exposure show an initial stable pattern that transitioned into the pattern of increasing effort seen during occlusions in air (FIGS. 2A-2B, tracheal pressure traces). Even clearer is the sustained high level of oxygen saturation in the oxygen-breathing rat that lasts about 30 seconds before the gradual decline with similar slope and endpoint to the air-breathing rat (FIGS. 2A-2B, pulse oximetry traces). Specific additional comparisons are illustrated in FIGS. 1A-1F.

Whereas oxygen exposure for five minutes prior to airway occlusion led to a 65% increase in the time to respiratory arrest (88.4 vs. 53.6 seconds), shorter oxygen exposures (30 seconds) were assessed and short exposures to more modest enrichment (50% oxygen / 50% air for 30 seconds). As shown in FIGS. 1A-1F, exposure to 30 seconds of oxygen immediately prior to airway occlusion (n=10) increased the time to respiratory arrest by 52% (81.6 vs. 53.6 seconds) and breath attempts (FIGS. 1E, 1F) that were not significantly different from the results obtained when exposure times were five minutes. When the concentration of oxygen was reduced by mixing oxygen and air 1:1, and applied for 30 seconds, the time to respiratory arrest trended to a higher value, increasing by 19% (63.9 vs. 53.6 seconds) compared with air (n=7 rats; p>0.05 after post-hoc correction for multiple comparisons, but p=0.02 in a straight comparison by t test) and the number of breath attempts was significantly increased.

The results of experiments on animals receiving either fluoxetine or saline (the saline control was important because of the large volume used to deliver drug) are shown in FIGS. 7A-7F. Fluoxetine delivered intraperitoneally and evaluated at one-hour post-injection produced an increase in the time tor respiratory arrest of 28% (63.8 vs. 49.9 seconds, n=8, 8), but the number of breath attempts was nearly equivalent (FIGS. 7D), indicating a lower rate of attempts to inspire.

When the impact of systemic fluoxetine on the pre-occlusion respiratory rate was evaluated, it was found that the main impact of fluoxetine was on altering the rate of breathing (FIGS. 7E, 7F). Tracheal pressure measures do not give information on tidal volumes.

The main action of fluoxetine on respiratory rate occurred pre-occlusion, suggesting that it did impact the respiratory brainstem as suggested by others (e.g., Bateman et al., "Serotonin Reuptake Inhibitors are Associated With Reduced Severity of Ictal Hypoxemia in Medically Refractory Partial Epilepsy," Epilepsia 51:2211-14 (2010); Blum, A.S., "Respiratory Physiology of Seizures," J. Clin. Neurophysiol. 26:309-15 (2009); Devinsky et al., "Sudden Unexpected Death in Epilepsy: Epidemiology, Mechanisms, and Prevention," Lancet Neural. 15:1075-88 (2016); Faingold et al., "Prevention of Seizure-Induced Sudden Death in a Chronic SUDEP Model by Semichronic Administration of a Selective Serotonin Reuptake Inhibitor," Epilepsy Behav. 22:186-90 (2011); Feng et al., "Abnormalities of Serotonergic Neurotransmission in Animal Models of SUDEP," Epilepsy Behav. 71:174-80 (2015); Massey et al., "Mechanisms of Sudden Unexpected Death in Epilepsy: The Pathway to Prevention," Nat. Rev. Neurol. 10:271-82 (2014); Richerson et al., "The Serotonin Axis: Shared Mechanisms in Seizures, Depression, and SUDEP," Epilepsia 52(Suppl 1):28-38 (2011); Tupal et al., "Evidence Supporting a Role of Serotonin in Modulation of Sudden Death Induced by Seizures in DBA/2 Mice," Epilepsia 47:21-6 (2006); and Zeng et al., "Fluoxetine Prevents Respiratory Arrest Without Enhancing Ventilation in DBA/1 Mice," Epilepsy Behav. 45:1-7 (2015).

The respiratory rate was significantly slowed by fluoxetine (FIGS. 7E, 7F). This lower rate of breath attempts during occlusion did not significantly alter the rate of desaturation, nor was there any measurable effect on the magnitudes of tracheal pressure changes during inspiration attempts. Also observed, but not statistically significant was a small difference in the minimum oxygen saturation (34% for fluoxetine group, 37% for saline group).

Interestingly, and consistent with the minimum oxygen saturation trend, it was more difficult to resuscitate animals in the SSRI group after respiratory arrest. Animals were resuscitated with bolus gas delivery as described previously (Villiere et al., " A Resuscitation Option for Upper Airway Occlusion Based on Bolus Transtracheal Lung Inflation," Laryngoscope Investig. Otolaryngol. 3:296-303 (2018). SSRI-treated animals required more attempts on average to effect resuscitation.

### Example 4 - Exposure to Oxygen and SSRI Prolonged Time to Respiratory Arrest.

A rat model was used to cause obstructive apnea with controlled airway occlusion to evaluate two interventions that have been shown to prevent death in animal models of sudden death in epilepsy. The times to respiratory arrest were determined in animals breathing oxygen vs. air, and animals treated with the selective serotonin receptor uptake inhibitor, fluoxetine. It was found that both oxygen exposure and fluoxetine (animals received either oxygen exposure or fluoxetine) significantly increased the time to respiratory arrest and, given that neither has been shown to alter seizure duration, these increases can account for the protection of either manipulation against death in sudden death models.

Importantly, it was found that 30 seconds of exposure to oxygen produced nearly the equivalent protection as five minutes exposure suggesting that oxygen exposure could start after a seizure starts. Experiments with 50% oxygen / 50% air mixtures indicate that the concentration needs to be above 50% to ensure times to respiratory arrest that are sufficiently long that they will always be longer than a period of seizure-induced airway occlusion.

Selective serotonin reuptake inhibitors, while instructive with regard to mechanism require impractical dosing and carry additional risk in the form of greater challenges for resuscitation.

### Example 5 - Relation of Oxygen Findings to Those in Mice.

Sufficient access to oxygen is the one manipulation shown herein to protect absolutely against death in any animal model of sudden death. That oxygen nearly doubled the time to respiratory arrest is not surprising given other experiences with oxygen, e.g., breath holding diving. As discussed previously (Nakase et al., "Laryngospasm, Central and Obstructive Apnea During Seizures: Defining Pathophysiology For Sudden Death in a Rat Model," Epilepsy Res. 128:126-139 (2016)), breathing oxygen prior to a breath holding dive prolonged the dive time by nearly two-fold. Based on experience, oxygen does not prolong seizure times. Prior to this disclosure, it was believed that there was not any evidence in any model where oxygen prolonged seizure times. The safety factor provided by oxygen with regard to the time to respiratory arrest completely explains why oxygen treatment ensures survival of 100% of animals (Venit et al., "Oxygenation Prevents Sudden Death in Seizure-Prone Mice," Epilepsia 45:993-6 (2004) and Willott et al., "Roles of Anoxia and Noise-Induced Hearing Loss in the Postictal Refractory Period for Audiogenic Seizures in Mice," J. Comp. Physiol. Psychol. 90:373-81 (1976).

### Example 6 - Relation of SSRI Findings to Those in Mice.

It was found that systemic fluoxetine (25 mg/kg given one hour prior to airway occlusion) significantly prolonged the time to respiratory arrest. The main effect of fluoxetine appeared to be a slowing of the respiratory rate, which occurred pre-occlusion and is consistent with prior work referred to herein on serotonergic contributions to respiration and protecting respiration in animals or people experiencing seizures. Fluoxetine may also have led to a significant or subtle hyperventilation effect to prolong the time to respiratory arrest similar to what has been described for breath holding (Godfrey et al., "The control of Breath Holding," Respir. Physiol. 5:385-400 (1968) and Marks et al., "Breath-Holding in Healthy and Pulmonary-Compromised Populations: Effects of Hyperventilation and Oxygen Inspiration," J. Magn. Reson. Imaging 7:595-7 (1997).

The doses of selective serotonergic reuptake inhibitors used in this model and in the mouse models are, however, medically impractical (roughly 10x clinical doses used for depression). More significantly, their use may permit respiratory arrest at lower overall oxygen saturations, which makes resuscitation more difficult.

### Example 7 - Hypoxia as Cause or Consequence of Apnea.

The mechanisms that have been proposed to account for death in the same mouse models that can be protected with oxygen exposure or SSRIs generally propose brainstem dysfunction caused by seizure activity (e.g., spreading depolarization) (Aiba et al., "Spreading Depolarization in the Brainstem Mediates Sudden Cardiorespiratory Arrest in Mouse SUDEP Models," Sci. Transl. Med. 7:282ra46 (2015), causing a form of central apnea, which drives desaturation and progresses to respiratory arrest. The protection afforded by SSRI pre-treatment has been viewed as consistent with this mechanism.

As described herein, the view has been that seizures "disrupt" brainstem activity, but by activating it as the seizures drive laryngospasm and thus obstructive apnea. The apnea persists as long as the laryngospasm persists, establishing a temporal competition for whether the seizure terminates before respiratory arrest or vice versa. In this view, the central form of apnea (i.e., respiratory arrest) is caused by the hypoxia, rather than a consequence.

Without limiting the system and methods disclosed herein to any particular mechanism of action, survival by audiogenic seizure prone mice after exposure to oxygen may be difficult to account for in the first view, but may be readily accounted for in the second. If oxygen exposure prevents death by preventing the brainstem dysfunction and central apnea of the first viewpoint, then it is unclear what seizure activity is directly responsible for. In this view, all of the seizure- driven events (including laryngospasm and obstructive apnea) still occur, but the temporal competition between seizure termination and respiratory arrest is eliminated because of the much longer times to respiratory arrest.

### Example 8 - Translation to SUDEP Prevention in Epilepsy Patients.

In accordance with the systems and methods described herein, protections against death may be translated into practical protections for epilepsy patients. Relatively brief access to an oxygen atmosphere just prior to airway occlusion led to a significant prolonged the time to respiratory arrest. The data indicates that as little as thirty seconds of oxygen immediately prior to airway closure can cause enough of an extension of the time to respiratory arrest that it would improve survival odds if seizure activity caused obstructive apnea. Thirty seconds would be within the time window of a seizure itself, suggesting that oxygen could be protective if supplied after a seizure starts.

### Example 9 - Portable System For Prevention of Death due to Seizure-Induced Laryngospasm or Other Transient Acute Upper Airway Obstruction.

The portable system described herein which prevents death is carried conveniently in a compact armband and pouch and is comprised of additional elements beyond the pouch and armband and is shown in FIGS. 8A-8B. In particular, the portable system includes an approximately 25-gram cartridge (201), the cartridge being filled with compressed oxygen (CO₂ cartridges weighing 25 grams hold about 861 cubic inches or 14.1 liter of gas). A connector (202) is also shown in FIG. 8A, and may be a simple connector to which the cartridge is connected, punctured for the controlled release of gas (similar to CO₂ cartridge-based bicycle tire repair connectors), as well as a fixed flow regulator with, for example, 8 liter/min or 4 liter/min flow options, simple indicator of flow or no flow, and attachment to a mask. A soft, flexible, collapsible oronasal mask (203) (made from silicone, for example) that has holes for a strap is shown in FIG. 8A, as well as an elastic strap (204) that will go around the back of the head and hold the mask over the mouth and nose. A pouch and armband that may be used in the portable system described herein is shown in FIG. 8B.

Operationally, the device may be activated and placed over the mouth and nose by an individual who believes that a seizure is about to occur. This can be based on the experience of aura, which many seizure patients report, another sense that a seizure is about to happen, or the report of a seizure prediction device. In this mode, the individual may activate the device and secure the strap to deliver oxygen even if the individual loses consciousness (which happens in generalized seizures). Alternatively, a bystander can activate the device and apply it to the face of a seizure patient experiencing a seizure. Alternatively, the device may include a wearable seizure detection device and the device may be activated upon detection of a seizure.

The flow of gas at 4 liters per minute may be maintained for about 3.5 minutes. A second cartridge in the kit will double the oxygen availability, or permit double the flow for the same total amount of time.

The delivery of oxygen, triggered approximately 30 seconds or more prior to complete airway occlusion due to laryngospasm or other cause, will delay any potential respiratory arrest and protect the individual from death due to laryngospasm-based airway obstruction. By preventing respiratory arrest, the device protects individuals who may not have a caregiver available for resuscitation if they experienced respiratory arrest.

The total time for the oxygen stream may be less than three minutes because the average duration of a tonic-clonic seizure is 1 to 3 minutes (*see, e.g*., Kiriakopoulos et al., "Tonic-Clonic Seizures," Epilepsy Foundation (2017).

At 4 liters per minute, inspired oxygen concentrations approaching 40% can be achieved, and at 8 liters per minute, inspired oxygen concentrations approaching 50% can be achieved. These concentrations were shown to be effective in preclinical (mouse and rat) studies where the time to respiratory arrest was measured or survival was measured (*see, e.g.,* FIG. 9, which shows fractions of inspired oxygen (FiO2) associated with different flow rates (Oxygen Delivery Flow Rates Cheat Sheet, available from NCLEXQuiz).

## Claims

1. A system for prevention of sudden death, said system comprising:
a wearable seizure detection device (114);
a base unit (100); and
a computer (106), said computer (106) configured to receive input from said seizure detection device (114)
**characterized in that**
the computer (106) is configured to trigger said base unit (100) to release oxygen under a condition effective to enrich an environmental oxygen level and prevent sudden death when said input indicates a seizure,
wherein the base unit (100) further comprises an attachment device (116) capable of providing a concentrated environmental oxygen level surrounding said attachment device (116).

2. The system of claim 1, wherein oxygen is released within about 30 seconds after a seizure is detected by said seizure detection device (114).

3. The system of any one of claims 1 or 2, wherein said condition is effective to enrich an environmental oxygen level to at least 50%.

4. The system of any one of claims 1 through 3, wherein oxygen is released under sustained conditions.

5. The system of any one of claims 1 through 4, wherein oxygen is released for a fixed duration of time, wherein in particular the fixed duration of time is for a period of between about 30 seconds to about 5 minutes.

6. The system of any one of claims 1 through 5, wherein the base unit (100) further comprises:
an oxygen tank (101);
a gas regulator (102);
an electronic valve (104);
a flow meter (108); and
a nozzle (112).

7. The system of claim 6, wherein said nozzle (112) is attached to said base unit (100) by a flexible tubing (110), wherein in particular said flexible tubing (110) has a length of more than about 1 foot.

8. The system of claim 1, wherein said attachment device (116) is a cone or a canopy.

9. The system of any one of claims 1 through 8, wherein said base unit (100) further comprises an oxygen sensor.

10. The system of any one of claims 1 through 9, wherein oxygen is released at a flow of between about 1 and about 250 standard cubic feet per minute.

11. The system of any one of claims 1 through 10, wherein the oxygen is released at a pressure of between about 1 psi and about 100 psi.

12. The system of any one of claims 1 through 11, wherein at least one of said input and said trigger is transmitted wirelessly.

13. The system of any one of claims 1 through 12, further comprising:
a remote control, said remote control capable of providing on demand oxygen release.

## Patentansprüche

1. System zur Prävention von plötzlichem Tod, das System umfassend:
eine tragbare Anfallsdetektionsvorrichtung (114);
eine Basiseinheit (100); und
einen Computer (106), wobei der Computer (106) dazu konfiguriert ist, eine Eingabe von der Anfallsdetektionsvorrichtung (114) zu empfangen,
**gekennzeichnet dadurch, dass**
der Computer (106) dazu konfiguriert ist, die Basiseinheit (100) dazu auszulösen, Sauerstoff unter einer Bedingung abzugeben, die dazu wirksam ist, ein Umgebungssauerstoffniveau anzureichern und einen plötzlichen Tod zu verhindern, wenn die Eingabe auf einen Anfall hinweist,
wobei die Basiseinheit (100) ferner eine Befestigungsvorrichtung (116) umfasst, die dazu fähig ist, ein konzentriertes Umgebungssauerstoffniveau, das die Befestigungsvorrichtung (116) umgibt, bereitzustellen.

2. System nach Anspruch 1, wobei Sauerstoff innerhalb von etwa 30 Sekunden abgegeben wird, nachdem ein Anfall von der Anfallsdetektionsvorrichtung (114) detektiert wird.

3. System nach einem der Ansprüche 1 oder 2, wobei die Bedingung dazu wirksam ist, ein Umgebungssauerstoffniveau auf mindestens 50 % anzureichern.

4. System nach einem der Ansprüche 1 bis 3, wobei Sauerstoff unter anhaltenden Bedingungen abgegeben wird.

5. System nach einem der Ansprüche 1 bis 4, wobei Sauerstoff für eine feste Zeitdauer abgegeben wird, wobei die feste Zeitdauer insbesondere ein Zeitraum von zwischen etwa 30 Sekunden bis etwa 5 Minuten ist.

6. System nach einem der Ansprüche 1 bis 5, wobei die Basiseinheit (100) ferner Folgendes umfasst:
einen Sauerstofftank (101);
einen Gasregler (102);
ein elektronisches Ventil (104);
einen Durchflussmesser (108); und
eine Düse (112).

7. System nach Anspruch 6, wobei die Düse (112) an der Basiseinheit (100) durch einen flexiblen Schlauch (110) befestigt ist, wobei der flexible Schlauch (110) insbesondere eine Länge von mehr als etwa 1 Fuß hat.

8. System nach Anspruch 1, wobei die Befestigungsvorrichtung (116) ein Trichter oder eine Kappe ist.

9. System nach einem der Ansprüche 1 bis 8, wobei die Basiseinheit (100) ferner einen Sauerstoffsensor umfasst.

10. System nach einem der Ansprüche 1 bis 9, wobei Sauerstoff mit einem Durchfluss von zwischen etwa 1 und etwa 250 Standardkubikfuß pro Minute abgegeben wird.

11. System nach einem der Ansprüche 1 bis 10, wobei der Sauerstoff bei einem Druck von zwischen etwa 1 psi bis etwa 100 psi abgegeben wird.

12. System nach einem der Ansprüche 1 bis 11, wobei mindestens eines von der Eingabe und dem Auslöser drahtlos übertragen wird.

13. System nach einem der Ansprüche 1 bis 12, ferner umfassend:
eine Fernsteuerung, wobei die Fernsteuerung fähig ist, auf Verlangen eine Sauerstoffabgabe bereitzustellen.

## Revendications

1. Système de prévention de la mort subite, ledit système comprenant :
un dispositif de détection de crise (114) portable ;
une unité de base (100) ; et
un ordinateur (106), ledit ordinateur (106) étant configuré pour recevoir une entrée provenant dudit dispositif de détection de crise (114),
**caractérisé en ce que**
l'ordinateur (106) est configuré pour déclencher ladite unité de base (100) afin de libérer de l'oxygène dans une condition efficace pour enrichir un niveau d'oxygène ambiant et prévenir la mort subite lorsque ladite entrée indique une crise,
dans lequel l'unité de base (100) comprend en outre un dispositif de fixation (116) capable de permettre un niveau d'oxygène ambiant autour dudit dispositif de fixation (116).

2. Système de la revendication 1, dans lequel de l'oxygène est libéré dans un délai d'environ 30 secondes après qu'une crise a été détectée par ledit dispositif de détection de crise (114).

3. Système de l'une quelconque des revendications 1 ou 2, dans lequel ladite condition est efficace pour enrichir un niveau d'oxygène ambiant à au moins 50 %.

4. Système de l'une quelconque des revendications 1 à 3, dans lequel l'oxygène est libéré dans des conditions maintenues.

5. Système de l'une quelconque des revendications 1 à 4, dans lequel l'oxygène est libéré pendant une durée fixe, dans lequel, en particulier, la durée fixe correspond à une période comprise entre environ 30 secondes et environ 5 minutes.

6. Système de l'une quelconque des revendications 1 à 5, dans lequel l'unité de base (100) comprend en outre :
un réservoir d'oxygène (101) ;
un régulateur de pression (102) ;
une vanne électronique (104) ;
un débitmètre (108) ; et
une buse (112).

7. Système de la revendication 6, dans lequel ladite buse (112) est fixée à ladite unité de base (100) au moyen d'un tuyau flexible (110), dans lequel, en particulier, ledit tuyau flexible (110) a une longueur supérieure à environ 1 pied.

8. Système de la revendication 1, dans lequel ledit dispositif de fixation (116) est un cône ou un auvent.

9. Système de l'une quelconque des revendications 1 à 8, dans lequel ladite unité de base (100) comprend en outre un capteur d'oxygène.

10. Système de l'une quelconque des revendications 1 à 9, dans lequel l'oxygène est libéré à un débit compris entre environ 1 et environ 250 pieds cubes normalisés par minute.

11. Système de l'une quelconque des revendications 1 à 10, dans lequel l'oxygène est libéré à une pression comprise entre environ 1 psi et environ 100 psi.

12. Système de l'une quelconque des revendications 1 à 11, dans lequel au moins l'un de ladite entrée et dudit déclenchement est transmis sans fil.

13. Système de l'une quelconque des revendications 1 à 12, comprenant en outre :
une télécommande, ladite télécommande étant capable de permettre la libération d'oxygène à la demande.
